(19) Europäisches Patentamt
European Patent Office
Office européen des brevets

(11) EP 2 878 365 B1

(12) EUROPÄISCHE PATENTSCHRIFT

(45) Veröffentlichungstag und Bekanntmachung des Hinweises auf die Patenterteilung:
12.04.2017 Patentblatt 2017/15

(51) Int Cl.:
B01F 7/00 (2006.01)          B01F 7/20 (2006.01)
B01F 15/00 (2006.01)         C12M 1/06 (2006.01)
C12M 1/107 (2006.01)

(21) Anmeldenummer: 14191283.2

(22) Anmeldetag: 31.10.2014

(54) **Rührwerk für einen Biogasfermenter**

Agitator for a biogas fermenter

Agitateur pour un digesteur à biogaz

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: 07.11.2013 DE 102013112255

(43) Veröffentlichungstag der Anmeldung:
**03.06.2015 Patentblatt 2015/23**

(73) Patentinhaber: **Agraferm Technologies AG**
**85276 Pfaffenhofen/Ilm (DE)**

(72) Erfinder:
• **Dr.-Ing. Kube, Jürgen**
**85276 Pfaffenhofen (DE)**
• **Mutke, Bernd**
**56729 Weiler (DE)**
• **Ellenrieder, Johannes**
**86424 Dinkelscherben (DE)**

(74) Vertreter: **Patronus IP Patent- und Rechtsanwälte**
**Neumarkter Strasse 18**
**81673 München (DE)**

(56) Entgegenhaltungen:
**EP-A1- 1 394 246          WO-A1-2008/101124**
**CH-A- 139 122            CH-A- 147 736**
**CN-U- 201 729 822         CN-U- 202 898 405**
**DE-A1-102005 050 927      DE-U1- 29 812 023**
**DE-U1-202004 004 101      DE-U1-202009 006 889**

**Beschreibung**

Die vorliegende Erfindung betrifft ein Rührwerksgestell und eine Rührwerksanordnung für einen Fermenter, einen Fermenter und eine Biogasanlage sowie Verfahren zum Erzeugen von Biogas und zum Umrüsten eines Fermenters.

In Deutschland sind seit der Einführung des Erneuerbare-Energien-Gesetz (EEG) in 2000 mehr als 7500 Biogasanlagen gebaut worden. Eine Vielzahl dieser Anlagen setzen Nawaro (nachwachsende Rohstoffe), also Energiepflanzensilagen wie Maissilage, Zuckerrüben und in geringeren Mengen auch Grassilage und Getreide-Ganzpflanzensilage (GPS) ein. Andere Länder in der EU haben ähnliche Anreize geschaffen, um Biogasanlagen zu bauen und zu betreiben und deren produzierte Energie in das Strom- oder Gasnetz einzuspeisen. Der große Zuwachs an Biogasanlagen der letzten Jahre hat lokal zu einem Steigen der Substratpreise, insbesondere der Silagepreise geführt. Anders als Körnergetreide oder Körnermais sind Silagen nicht weltweit, sondern nur lokal handelbar. Sie haben einen hohen Wassergehalt und verderben leicht. Eine lokale Konzentration von Biogasanlagen führt daher zu einem Ansteigen der Substratpreise, insbesondere der Maissilagepreise. Es besteht am Markt daher ein großes Interesse, Biogasanlagen in die Lage zu versetzen, andere Substrate als Mais und Gülle einzusetzen. Die Umstellung des Substratmix hat jedoch Auswirkungen auf den Betrieb der Anlage, insbesondere auf die Viskosität des Gärsubstrates und die Durchmischung des Fermenterinhaltes.

Zur Durchmischung von Biogasanlagen sind beispielsweise Propellerrührwerke bekannt. Propellerrührwerke werden zur turbulenten Durchmischung eingesetzt. Die turbulente Durchmischung zeichnet sich durch eine konstante Newton-Zahl aus. Die Newton-Zahl ist definiert als:

$$\mathrm{Ne} = \frac{P_W}{n^3 \cdot d^5 \cdot \rho}$$

mit

Ne   [-] Newton-Zahl,
$P_W$   [kW] Wellenleistung,
$n$   [rpm] Umdrehungszahl des Rührwerks,
$d$   [m] Rührwerksdurchmesser, und
$\rho$   [kg/m$^3$] Dichte des Mediums.

Eine turbulente Durchmischung ist erkennbar an dem Zusammenhang

$$P_W \sim n^3 \cdot d^5$$

Die Leistungsaufnahme ist unabhängig von der Viskosität des Fermenterinhalts. Bekannt sind beispielsweise Rührwerke, die einen Durchmesser von ca. 60 cm aufweisen und mit ca. 400 U/min betrieben werden. Dabei werden Antriebsleistungen von ca. 8 bis 15 W pro m$^3$ Fermentervolumen installiert, wobei ca. 10 W/m$^3$ im Dauerbetrieb benötigt werden. Wird eine turbulente Durchmischung des Behälters angestrebt, müssen die Rührwerke so ausgewählt, dimensioniert und angeordnet werden, dass sich ein möglichst großer Bereich mit einer Mindestgeschwindigkeit von etwa 1 bis 5 cm/s einstellt. Bei dieser Geschwindigkeit kommt es nicht mehr zu einem Entmischen des Gärsubstrates. Die Rührwerkskavernen verbinden sich dann zu einer einzigen großen Kaverne und der Fermenter ist weitgehend ideal durchmischt. Die Scherrate an den Propellerrührern liegt bei Werten von ca. 50 s$^{-1}$. Bei den Propellerrührwerken haben sich in letzter Zeit größere Rührwerksdurchmesser (bis zu 2 m) und geringere Drehzahlen durchgesetzt. Diese Rührwerke verringern den Rührenergiebedarf, können aber nur in niederviskosen Fluiden eingesetzt werden.

Die Propellerrührwerke können als Tauchmotorrührwerke oder Stabmixer ausgeführt werden. Bei Stabmixern liegt der Antriebsmotor außerhalb des Behälters und die Rührwelle wird durch die Behälterwand oder seltener durch die Behälterdecke geführt. Tauchmotorrührwerke weisen einen elektrischen oder hydraulischen Antrieb auf. Zur Energieversorgung muss eine Stromversorgung oder eine Hydraulikleitung von außen in den Behälter zu den Rührwerken geführt werden. Die Energieleitung stellt dabei häufig ein Problem im Betrieb der Biogasanlage dar. Stromführende Kabel scheuern an der Eintrittsstelle in den Fermenter durch und können dort für Funken sorgen, was in der Vergangenheit zu Bränden auf Biogasanlagen geführt hat. Bei Hydraulikleitungen kann ein Platzen der Leitung zur Kontamination des Fermenterinhalts führen. Da der Fermenterinhalt später als Dünger verwendet wird, kann eine Kontamination mit Hydrauliköl eine Entsorgung des gesamten Fermenterinhalts notwendig machen. Biogasfermenter mit Propellerrührwerken weisen meist eine Folienabdeckung auf, die z.B. flexibel oder starr einschalig, als Doppelmembran oder Tragluft-Doppelmembran ausgeführt ist.

Neben Fermentern mit Propellerrührwerken werden auch zentral gerührte Behälter auf Biogasanlagen eingesetzt. In diesem Fall kommen meist klassische Balkenrührer zum Einsatz. Sie werden von oben in der Behälter eingeführt und weisen ein Bodenlager auf oder sind freifliegend (nur einseitig gelagert) aufgehängt. Auch diese Rührwerke können in klassischen Biogasanlagen nur im nieder- bis mittelviskosen Bereichen eingesetzt werden.

Hochviskose Gärsubstrate wie sie speziell bei Mist-, Gras- oder GPS-haltigen Substratmischungen anfallen, werden oft mit langsamlaufenden Paddelrührwerken umgewälzt. Diese werden horizontal, vertikal oder als Schrägachs-Rührwerke ausgeführt. Horizontale und Schrägachsrührwerke werden durch die Behälterwand geführt. Der Rührwerksmotor sitzt dabei außen und führt die Drehmomente an die Behälterwand oder über eine Drehmomentenstütze an den Boden bzw. ein Fundament ab. Ein Vorteil der horizontalen Rührwerke ist, dass sie mit ihren Paddeln eine eventuell vorhandene Schwimmschicht zerschlagen. Dieser Vorteil wird mit dem Nachteil erkauft, dass horizontale Rührwerke nicht dazu beitragen, den Behälterinhalt als Ganzes in eine tangentiale Rotationsbewegung zu versetzen. Horizontale Paddelrührwerke werden daher häufig mit Propellerrührwerken kombiniert, was jedoch wieder eine geringe Viskosität voraussetzt. Eine Sonderform der horizontalen Paddelrührwerke ist das horizontale Haspelrührwerk in rechteckigen Fermentern. Vertikale Paddel-Rührwerke werden von oben durch die Behälterdecke geführt. Ihr Drehmoment muss über die Decke abgeführt werden. Weisen alle vertikalen Paddelrührwerke dieselbe Drehrichtung auf, stellt sich im Behälter eine langsame Rotation ein. Durch den Einbau mehrerer Rührwerke und den wechselweisen kombinierten Betrieb können eventuelle Totzonen gezielt durchströmt werden. Bei der Verwendung von langsamlaufenden Paddelrührwerken liegt meist eine laminare Durchmischung vor.

Eine laminare Durchmischung ist gekennzeichnet durch den Zusammenhang

$$\mathrm{Ne} \sim \frac{1}{\mathrm{Re}}$$

mit der Definitionsgleichung der Reynoldszahl

$$\mathrm{Re} = \frac{n \cdot d^2 \cdot \rho}{\eta}.$$

Die Reynoldszahl ist somit abhängig von der dynamischen Viskosität $\eta$. Im laminaren Bereich existiert also ein Zusammenhang zwischen Wellenleistung des Rührwerks und der Viskosität des Fermenterinhalts. Der genaue Zusammenhang ist abhängig von der Rheologie des Gärsubstrats. Es hat sich gezeigt, dass bei landwirtschaftlichen Biogasanlagen das Gärsubstrat bei geringen Trockenrückständen strukturviskos ist. Steigt der Trockenrückstand TR des Gärsubstrats auf Werte über ungefähr 10 % (substratabhängig) ändert sich das Fließverhalten hin zu einem Bingham- oder Herschel-Bulkley-Fluid. Diese Fluide zeichnen sich durch eine Grenzschubspannung aus, die überwunden werden muss, um ein Fließen des Fluides zu ermöglichen. Der Zusammenhang zwischen Wellenleistung, Drehzahl, Durchmesser sowie den rheologischen Eigenschaften des Fluids bei laminarer Durchmischung lauten bei Bingham-Fluiden:

$$\eta = \eta_\infty + \frac{\tau_0}{\dot{\gamma}},$$

$$P \sim \eta_\infty \cdot n^2 \cdot d^3 + \frac{\tau_0}{K_{MO}} \cdot n \cdot d^3$$

und bei strukturviskosen Fluiden:

$$\eta = K \cdot \dot{\gamma}^{(m-1)},$$

$$P \sim n^{(1+m)} \cdot d^3$$

mit

$\eta_\infty$      [Pas] Viskosität bei unendlich hoher Scherrate,
$\tau_0$      [Pa] Fließgrenze,
$\dot{\gamma}$      [s$^{-1}$] Scherrate,
$K_{MO}$      [-] Metzner-Otto-Konstante des Rührwerks,
$K$      [Pa·s$^m$] Konsistenzfaktor, und
$m$      [-] Fließindex.

Beide Fließverhalten lassen sich grob mit der folgenden Formel wiedergeben:

$$P \sim n^{1..2} \cdot d^3 .$$

Anders als bei der turbulenten Durchmischung ist bei der laminaren Durchmischung keine möglichst hohe mittlere Geschwindigkeit im Behälter notwendig. Aufgrund der hohen Viskosität des Gärsubstrates kommt es nicht mehr zu einem Entmischen des Gärsubstrates. Speziell bei Gärsubstraten mit Fließgrenzen sinken Substratpartikel nicht mehr zu Boden, wenn sie einmal in das Gärsubstrat eingerührt sind. Umgekehrt können aber auch die gebildeten Gasblasen ohne Energieeintrag von außen nicht mehr aus dem Gärsubstrat entweichen. Die laminaren Rührwerke stellen daher auch die Entgasung des Gärsubstrates sicher; auf der Rückseite der Rührwerke sammeln sich die Gasblasen und erreichen Durchmesser, die ein Aufsteigen der Gasblasen ermöglichen. Die laminare Durchmischung hat gegenüber der turbulenten Durchmischung den Vorteil, dass höherviskose Gärsubstrate durchmischt werden können. Insbesondere können Gülle, Wasser oder Prozesswasser zum Verdünnen des Fermenterinhaltes reduziert oder weggelassen werden, was dazu führt, dass die Verweilzeit im Fermenter ansteigt, bzw. mehr Substrat bei gleicher Fermentergröße und Verweilzeit eingesetzt werden kann. Dies wird sichtbar an einer steigenden Raumbelastung.

Die Viskosität von Gärsubstraten ist von einer Vielzahl von Parametern abhängig. Der wichtigste Parameter ist der Trockenrückstand des Gärsubstrates. Es besteht ein exponentieller Zusammenhang zwischen Trockenrückstand und Viskosität. Häufig wird aus diesem Grund Gülle oder Prozesswasser aus der Gärrestseparation in Anlagen gegeben, um den Trockenrückstand im Fermenter abzusenken und die Anlage zu verdünnen. Dies hat jedoch den Nachteil, dass die Verweilzeit im Fermenter und damit auch der Abbaugrad des Substrates drastisch sinken. Der zweitwichtigste Einflussparameter ist die Zusammensetzung der Substratmischung. Anlagen, die Gras und GPS einsetzen, zeigen eine höhere Viskosität als Anlagen, die hauptsächlich Mais und Gülle einsetzen. Bei GPS kann dieser Effekt auf die Schleimstoffe des Getreides zurückgeführt werden. Bei Gras kommt es zu einem Verhaken der Grasfasern untereinander im Fermenter. Einige Substrate zeigen nach der Vergärung überraschender Weise auch noch bei hohen Trockenrückständen eine geringe Viskosität (z.B. Mist, insbesondere Pferdemist). Ein weiterer wichtiger Einflussparameter ist die Faserlänge des eingesetzten Substrates. Speziell beim Einsatz von Gras reduziert eine kurze Faserlänge die Viskosität des Fermentermaterials ganz erheblich. Weitere Einflussparameter sind Temperatur und Partikelgrößenverteilung. Typische Gärsubstrate aus laminar durchmischten Biogasanlagen wurden mit einem Rotationsviskosimeter vermessen. Die rheologischen Eigenschaften der Gärsubstrate sind in Tabelle 1 für drei ausgewählte Substratmischungen (Mix A, B, und C) aus laminar gerührten Biogasanlagen dargestellt.

**Tabelle 1**

| Mix Nr. | Substrate | TR | $\tau_0$ [Pa] | $\eta_\infty$ [m Pas] |
|---------|-----------|-----|---------------|------------------------|
| (A) | 80% Maissilage, 10% Grassilage, 10% GPS | 11% | 5 | 40 |
| (B) | 33% Maissilage, 33%, Grassilage, 33% GPS | 12% | 23 | 6,7 |
| (C) | 20% Maissilage, 60% Grassilage, 20% Hühnertrockenkot | 15% | 18 | 73 |

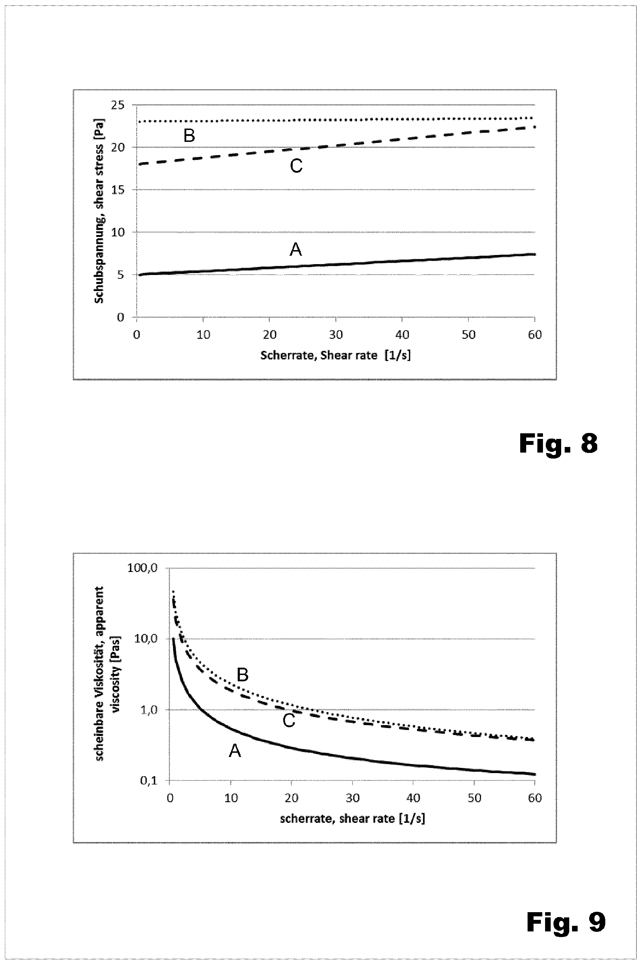

Fig. 8 zeigt ein Diagramm gemessener Schubspannungen in Abhängigkeit von der Scherrate. Die Gärsubstrate zeigen das Fließverhalten eines Bingham-Fluides. Aus CFD-Berechnungen ist den Erfindern bekannt, dass die Scherrate in laminar gerührten Biogasanlagen bei Werten unter 0,5 s$^{-1}$ liegt. Die Viskosität des Gärsubstrates ist bei Bingham-Fluiden somit nur von der Grenzschubspannung abhängig. Der scherratenabhängige Anteil der Schubspannung spielt in diesem Einsatzfall eine im Wesentlichen verschwindende Rolle. Eine Unterscheidung zwischen Bingham- und Herschel-Bulkley-Fluiden ist daher hier nicht notwendig. Die Viskosität in diesem Bereich ist in Fig. 9 in Form eines Diagramms in Abhängigkeit von der Scherrate dargestellt.

Behälter, speziell Fermenter, in Biogasanlagen werden als Beton- oder Stahlbehälter ausgeführt. Betonbehälter können so ausgeführt werden, dass sie die Schubkräfte und Drehmomente der Rührwerke aufnehmen können. Oftmals werden die Behälter aber nur auf die hydrostatische Belastung durch das Gärsubstrat ausgelegt. Die Rührwerkskräfte und -momente werden vernachlässigt oder müssen aus dem Behälter heraus in den Boden abgeleitet werden. Einige Behälter weisen eine Betondecke auf, in die vertikale Paddelmixer oder Stabmixer eingebaut werden können. Es ist jedoch nicht immer möglich, einen Betonbehälter im Nachhinein mit einer Betondecke auszurüsten. Die Gewichtskraft der Betondecke muss an der Wand und an der Mittelstütze abgeleitet werden, was zu erhöhten Druckbelastungen und damit zu einem Setzen des Bodens in diesem Bereichen führen kann. Stahl-Behälter können im Allgemeinen nicht nachträglich mit einer stabilen Decke ausgeführt werden, welche die Drehmomente von laminaren vertikalen Rührwerken aufnehmen kann.

Weitere Informationen über die aktuelle Rührwerkstechnik in Biogasanlagen sind in dem Positionspapier "Empfehlungen für die Auswahl von Rührwerken für Gärbehälter" des Biogas Forum Bayern von 2010 (http://www.biogas-forum-bayern.de/publikationen/Empfehlungen.fuer.die.Auswahl.von.Ruehrwerken.fuer.Gaerbeh aelter.pdf, heruntergeladen am 24.10.2013) und in dem "Leitfaden Biogas" aus der Mediathek der Fachagentur für Nachwachsende Rohstoffe (FNR) (publiziert in mehreren Sprachen, u.a. http://mediathek.fnr.de/media/downloadable/files/samples/g/u/guide_biogas_engl_20 12.pdf, heruntergeladen am 24.10.2013) zu finden.

US 4,982,373 betrifft Rührvorrichtungen für Flüssigkeiten und/oder Gase in einem abgeschlossenen Raum. Ziel der Autoren dieser Druckschrift war es, eine Rührvorrichtung bereitzustellen, wobei die Rührwelle nicht durch Abschnitte eines geschlossenen Raumes hindurchläuft, um so mögliche, undichte Stellen zu vermeiden. Deswegen offenbart die US 4,982,373 eine Rührvorrichtung, welche zusammen mit ihrem Antrieb komplett in dem geschlossenen Raum untergebracht ist, wobei sich die gesamte Rührvorrichtung innerhalb dieser Kammer befindet.

In der DE 10 2005 041 798 A1 ist ein entnehmbares, vertikales Paddelrührwerk beschrieben, welches eine laminare Durchmischung einer Biogasanlage ermöglicht. Die Drehmomente und Kräfte des Rührwerks werden über eine Betondecke abgeführt. Die Rührwerksmotoren befinden sich oberhalb der Fermenterdecke.

In der DE 10 2010 053 242 A1 ist ein Fermenter beschrieben, der ohne den Einsatz einer Betondecke laminar gerührt werden kann. Das vertikale Paddelrührwerk wird mit einem Tragwerk gehalten, das oberhalb des maximalen Füllstands verläuft. Die Rührwerkswelle wird auf die Außenseite des Foliendaches geführt. Der Antrieb ist außerhalb des Foliendaches auf dem Tragwerk angeordnet. Diese Rührwerksanordnung erfordert ein Durchstoßen des Foliendaches an mindestens einem Punkt. Dieser Punkt stellt einen Fixpunkt dar und schränkt die Bewegung des Foliendaches ein, so dass die Speicherkapazität des Gasspeichers reduziert wird. Zudem durchstößt auch das Tragwerk das Foliendach. An der Verbindung zwischen Tragwerk und Foliendach muss eine Dichtung installiert und gewartet werden. Die Stelle ist schwer zu erreichen. Zudem stellen die Dichtungen häufig einen Tiefpunkt dar, in dem sich Wasser und Schnee sammelt, was zu einer Beschädigung des Gasspeichers führen kann. Im Gegensatz zur vorliegenden Erfindung befinden sich die Rührwerksmotoren oberhalb der Folienabdeckung und die Rührwerkskräfte und -momente werden in die Behälterwand eingetragen.

Die DE 20 2006 004 982 U1 offenbart ein horizontales Paddelrührwerk, bei welchem die drehbare Welle im Behälter mit einem Gestell gelagert wird. Das Lager befindet sich am Gestell und kann radiale und im geringen Maße auch axiale Kräfte aufnehmen. Aufgrund der Lagerung können keine Drehmomente in das Gestell abgetragen werden. Die Rührwerksdrehmomente werden außerhalb des Behälters an die Behälterwand abgetragen. Alternativ ist auch die Einleitung der Drehmomente in den Boden außerhalb des Behälters möglich.

In der DE 201 11 596 U1 ist eine Rührwerksanordnung von Tauchmotorrührwerken offenbart. Dabei ist ein Tauchmotorrührwerk mit einer vertikalen Achse gezeigt. Das Tauchmotorrührwerk ist an einem Tauchmotorrührwerksmast aufgehängt, der die Kräfte und Momente der Rührwerke aufnimmt und teilweise in den Boden ableitet. Das Tauchmotorrührwerk weist ein Propellerrührwerk und ein Lager im Motorgehäuse auf. Es wird zur turbulenten Durchmischung verwendet.

Die DE 10 2010 014 239 A1 beschreibt ein Verfahren zum Betreiben eines Biogasfermenters. Der Fermenter wird mit horizontalen Rührwerken gerührt, die ihre Kräfte und Momente vollständig in die Bodenplatte eintragen. Die Anordnung der Rührwerke geschieht so, dass sich eine gemeinsame Mischzone ausbildet. Die Rührwerke erzeugen eine zumeist horizontale Bewegung.

Die JP 2011-088034 A offenbart einen Rührer für einen offenen Behälter. Das Drehmoment des Rührers wird über einen Träger in den Behälterboden eingetragen. Der Träger befindet sich außerhalb des Behälters. Der Behälter ist mobil.

In der WO 2009/018971 A1 wird eine Methode beschrieben, um bei langen unverzweigten doppelwandigen Rohren den Leckage-Ort des inneren Rohres über die Transportzeit des äußeren Fluides zu detektieren.

Die WO 001995031353 A1 beschreibt ein allgemeines Leckerkennungsverfahren für doppelwandige Hydraulikleitungen mit einer Durchfluss- und Drehzahlüberwachung der Hydraulikpumpe.

DE 20 2004 004 101 U1 offenbart ein Rührwerksgestell für einen Fermenter gemäß dem Oberbegriff des Anspruchs 1.

Der Erfindung liegt die Aufgabe zu Grunde, die Nachteile im Stand der Technik wenigstens teilweise zu vermeiden. Insbesondere ist eine Aufgabe der Erfindung, Bestandsbiogasanlagen im Hinblick auf ihren Substratmix so zu erweitern, dass andere Substrate mit geringeren Kosten eingesetzt werden können, insbesondere Substrate, bei deren Vergärung ein zähflüssigeres Gärsubstrat entsteht. Eine weitergehende Aufgabe der Erfindung besteht darin, Fermentationsbehälter mit Folienabdeckung auf einer Biogasanlage, die für ein turbulentes Regime ausgelegt war, so umzurüsten, dass der Behälter sicher laminar durchmischt werden kann, wobei bevorzugt die Behälterabdeckung als ein konventioneller Folien-Gasspeicher ausgeführt werden kann und keine Naht- und Dichtungsstellen zwischen Folienabdeckung und Rührwerk entstehen, sodass der bestehende Foliengasspeicher weiter genutzt werden kann und ungünstig zu erreichende Wartungsstellen zwischen der Folienabdeckung und einem eventuell vorhandenem Tragwerk für die Rührwerke vermieden werden können.

Die vorstehend genannten Aufgaben werden wenigstens in Teilaspekten durch die Merkmale der unabhängigen Ansprüche gelöst. Bevorzugte Ausführungsformen und vorteilhafte Weiterbildungen der Erfindung sind in den Unteransprüchen angegeben. Nach einem Gesichtspunkt der vorliegenden Erfindung wird ein Rührwerksgestell für einen Fermenter, mit den Merkmalen des Anspruchs 1 vorgeschlagen.

Als ein Fermenter wird im Sinne der Erfindung ein vorzugsweise beheizbarer Behälter insbesondere auf einer Biogasanlage zur Erzeugung von Biogas aus Substrat durch Fermentation des Substrats verstanden. In Anlagen mit mehreren Gärstufen kann jede Gärstufe als Fermenter im Sinne der Erfindung verstanden werden. So wird beispielsweise auch ein sog. Nachgärer, der einem primären Fermenter oder Hauptfermenter nachgeschaltet ist, als Fermenter im Sinne der Erfindung verstanden. Dabei weist jedenfalls ein Hauptfermenter vorzugsweise, aber nicht notwendig, einen nahezu konstanten Füllstand an Substrat auf.

Das erfindungsgemäße Rührwerksgestell erlaubt die Installation eines oder mehrerer vertikaler, insbesondere frei auf einer Bodenplatte stehender Rührwerke in einen Fermenter, ohne ein Dach des Fermenters zu durchstoßen. Hierdurch können Dachöffnungen und hiermit verbundene Abdichtungsprobleme vermieden werden. Das ist insbesondere bei Foliendächern bedeutsam, denen eine gewisse Eigenbeweglichkeit immanent ist. Dadurch können auch Leckverluste vermindert werden, sodass der Ertrag und die Effizienz der Anlage gesteigert und die Belastung der Atmosphäre mit Treibhausgasen (Methan) verringert werden kann. Im Übrigen kann ein Foliendach im Wesentlichen kein Moment aufnehmen, sodass die Verwendung von vertikalen Rührwerken in einem Behälter mit Foliendach durch die Erfindung erst ermöglicht wird. Durch eine vertikale Anordnung der Rührwerke kann der Fermenter auch so betrieben werden, dass das Substrat langsam laminar umgewälzt werden kann. Daher können auch dickflüssigere Substrate eingesetzt werden. Ein dickflüssigeres Substrat weist einen geringeren Wasseranteil und mehr biologisch abbaubares Material auf. Daher kann auch eine höhere Effizienz bei gleicher Fermentergröße erzielt werden. Es können auch andere Substrate mit einem höheren Feststoffanteil eingesetzt werden. Es versteht sich, dass das Rührwerksgestell auch zur Installation schräger oder horizontaler Rührwerke ausgebildet sein kann. Der Vorteil verminderter Leckage kann auch in diesem Fall erzielt werden, da keine beweglichen Wellen durch die Fermenterwandung hindurchgeführt werden müssen, was die Abdichtung und Wartung des Fermenters erheblich vereinfacht.

Als ein Rahmen wird im Sinne der Erfindung eine starre bzw. im Wesentlichen starre Raumform verstanden, welche die Anbringung weiterer konstruktiver Bauelemente ermöglicht. Der Rahmen kann aus einem Stück hergestellt sein, insbesondere beispielsweise aus einer Platte, wenn nur eine einzige Lageraufnahme (und Motoraufnahme) vorgesehen ist, oder aus einem Träger bzw. Balken, wenn nur zwei Lageraufnahmen (und Motoraufnahmen) vorgesehen sind. Bei mehr als zwei aufzunehmenden Rührwerken kann der Rahmen insbesondere ein Strebwerk mit mehreren miteinander verbundenen Streben sein, welches die mehreren Lageraufnahmen und zugeordneten Antriebsaufnahmen miteinander trägt oder aufweist. Einzelne Bestandteile, beispielsweise Streben, des Rahmens können miteinander verbunden sein, etwa durch Schrauben, Nieten oder dergleichen, oder, etwa durch Schweißen, Verkleben oder dergleichen, zu einem Ganzen gefügt sein. In einer Abwandlung kann der Rahmen als ein Fachwerk ausgebildet sein, d.h. sich aus Stäben zusammensetzen, die im Wesentlichen nur durch Zug- und Druckkräfte beansprucht werden und deren Knoten im Wesentlichen momentenfrei sind. Um die Drehmomente der Rührwerke aus dem Gestell abzuführen, ist bei dieser Abwandlung mindestens ein Träger notwendig, der auch mit einem Biege- bzw. Torsionsmoment beaufschlagbar ist.

Eine Lageraufnahme ist im Sinne der Erfindung eine konstruktiv umgesetzte Schnittstelle zur Anbringung eines Lagers, und eine Antriebsaufnahme ist im Sinne der Erfindung eine konstruktiv umgesetzte Schnittstelle zur Anbringung eines Antriebs. Eine Schnittstelle kann in den Rahmen integriert oder daran befestigt sein. Insbesondere kann eine Schnittstelle eine vorgegebene und vorbereitete Befestigungsstelle sein, die beispielsweise als Flanschplatte, Knotenblech, Lagerbock, Lochblech, Lochwinkel, Ausnehmung, Anordnung von Durchgangsbohrungen, Gewindebohrungen, Stehbolzen oder dergleichen oder auf andere Weise ausgebildet ist. Die Lageraufnahme und die Antriebsaufnahme können durch ein und dasselbe Bauelement oder durch verschiedene Bauelemente ausgebildet sein.

Ein Fermenterkörper ist im Sinne der Erfindung eine bauliche Hülle des Fermenters, welche im Allgemeinen eine Bodenplatte, eine Seitenwand (vorzugsweise rund, gegebenenfalls auch oval oder polygonal) und ein Dach umfassen kann. Eine Verankerung des Rahmens relativ zu dem Fermenterkörper bedeutet im Sinne der Erfindung, dass der Rahmen mit Rührwelle/-n und Rührantrieb/-en in der Einbausituation in seiner Position innerhalb des Fermenterkörpers unbeweglich bzw. im Wesentlichen unbeweglich montiert bzw. abgestützt ist. Die Verankerungseinrichtung kann den Rahmen innerhalb des Behälterkörpers gegen die Bodenplatte abstützen und kann ebenfalls ein Strebwerk (oder Fachwerk) aufweisen. Alternativ oder zusätzlich kann die Verankerungseinrichtung auch eine Verbindung zwischen dem Rahmen und der Behälterwand vorsehen, falls die Wand Kräfte aufnehmen kann. Es kann, aber nicht Teil der vorliegenden Erfindung, zusätzlich oder alternativ auch vorgesehen sein, dass die Verankerungseinrichtung sich durch die Seitenwand des Behälterkörpers hindurch erstreckt und außerhalb des Behälterkörpers am Boden, an Baukörpern oder anderen Fixpunkten befestigt ist; in diesem Fall können Zuleitungen zu dem/den Rührantrieb/-en leicht an der Verankerungseinrichtung befestigt werden oder ggf. auch im Inneren von deren Strebenprofilen geführt werden. Das Rührwerksgestell bzw. seine Bestandteile kann bzw. können grundsätzlich aus jedem geeigneten, insbesondere ausreichend stabilen, temperaturbeständigen und gegenüber den Bestandteilen eines Gärsubstrat und/oder einer Biogas-Atmosphäre reaktionsträgen Material, vorzugsweise aus einer Stahlsorte, wie etwa Baustahl oder dergleichen, einem anderen Metall oder einem Kunststoff einschließlich eines Faserverbundwerkstoffs hergestellt sein; sie können auch vergütet oder beschichtet sein, um die gewünschte chemische Beständigkeit bereitzustellen. Wenn der/die Rührantrieb/-e oberhalb einer maximalen Füllhöhe angeordnet sind, ist die Abdichtung der Antriebe und Zuleitungen weniger aufwändig. Es ist aber auch ein getauchter Betrieb möglich.

Im Rahmen der vorliegenden Erfindung ist die Verankerungseinrichtung ausgebildet, um an einer Bodenplatte und/oder an einer Seitenwand des Fermenterkörpers befestigt zu werden. Bei dieser Ausführungsform werden Antriebsmomente und obere Lagerkräfte entweder ausschließlich in die Bodenplatte oder ausschließlich in die Seitenwand oder anteilig in Bodenplatte und Seitenwand eingeleitet. Eine Aufteilung kann beispielsweise derart vorgesehen sein, dass horizontale Kräfte bevorzugt in die Seitenwand geleitet werden und vertikale Kräfte bevorzugt in die Bodenplatte geleitet werden. Eine Krafteinleitung in die Seitenwand erfordert dabei, dass die Wand entsprechende Kräfte aufnehmen kann. Bei einer Abstützung an der Seitenwand können vorzugsweise zwei unparallele horizontale Streben vorgesehen sein, um Momente als Zug-/Druckkräfte übertragen zu können. Dadurch können punktuelle Momenteeinleitungen in die Behälterwandung vermieden werden. In allen Fällen können Drehmomente des Motors somit über eine starre Konstruktion zuverlässig an die Bodenplatte und/oder Seitenwand des Behälters abgetragen werden.

Es versteht sich, dass die Geometrie des Rührwerksgestells, insbesondere die Anordnung, Lage und Ausrichtung der Lageraufnahmen, Antriebsaufnahmen und Verankerungsstellen, so ausgelegt ist, dass auch die Merkmale und Vorteile der nachstehend beschriebenen weiteren Erfindungsgesichtspunkte und ihrer Abwandlungen, Varianten und Alternativen verwirklicht werden können, sodass die Merkmale der nachstehend beschriebenen weiteren Erfindungsgesichtspunkte und ihrer Abwandlungen, Varianten und Alternativen gleichzeitig Abwandlungen, Varianten und Alternativen des vorliegenden Erfindungsgesichtspunkts definieren.

In einer bevorzugten Ausführungsform ist die Lageraufnahme zur Lagerung einer vertikalen Rührwelle ausgebildet, wobei die Lageraufnahme zur Aufnahme eines oberen Rührwellenlagers der Rührwelle ausgebildet ist. Das obere Rührwellenlager befindet sich im oberen Endbereich der Rührwelle. Die Rührwelle ist insbesondere mittels eines unteren vor allem lösbaren Rührwellenlagers am unteren Endbereich der Rührwelle gelagert, wobei das untere Rührwellenlager an einem Boden des Fermenters anordbar sein kann. Die Rührwelle erstreckt sich vom unteren Rührwellenlager bis in einen Bereich oberhalb des maximalen Füllstandes des Fermenters.

Im Rahmen der vorliegenden Erfindung ist das untere Rührwellenlager am unteren Endbereich der Rührwelle lösbar an einem Boden des Fermenterkörpers anordbar, so dass die Rührwelle angehoben werden kann. Dadurch kann die gesamte Rührwelle mit dem kompletten Rührwerk leicht aus dem Fermenter entfernt und ausgetauscht oder gewartet werden.

Ein weiterer Gesichtspunkt der vorliegenden Erfindung betrifft eine Rührwerksanordnung für einen Fermenter, mit den Merkmalen des Anspruchs 4.

Da die erfindungsgemäße Rührwerksanordnung das erfindungsgemäße Rührwerksgestell aufweist, werden die im Zusammenhang mit dem Rührwerksgestell beschriebenen Vorteile auch durch die Rührwerksanordnung verwirklicht.

In einer bevorzugten Ausführungsform der Rührwerksanordnung ist die Lageraufnahme zur Lagerung einer vertikalen Rührwelle vorgesehen, wobei die Lageraufnahme zur Aufnahme eines oberen Rührwellenlagers der Rührwelle ausgebildet ist. Das obere Rührwellenlager befindet sich im oberen Endbereich der Rührwelle. Die Rührwerksanordnung weist ein unteres Rührwellenlager auf, das am unteren Endbereich der Rührwelle angreift, wobei das untere Rührwellenlager lösbar ausgebildet ist und ein an einem Boden des Fermenters anordbares bzw. befestigbares Element aufweist. Die Rührwelle erstreckt sich vorzugsweise vom unteren Rührwellenlager bis in einen Bereich oberhalb des maximalen Füllstandes des Fermenters.

In einer bevorzugten Ausführungsform ist das untere Rührwellenlager am unteren Endbereich der Rührwelle lösbar an der Rührwelle angebracht. Dies ermöglicht ein leichtes Austauschen des Lagers bei Bedarf.

Grundsätzlich kann mit einem einzigen Rührwerk der Inhalt eines Fermenters wirksam durchmischt werden. Aus Gründen der Redundanz ist es vorteilhaft, wenn wenigstens zwei Rührwerke vorhanden sind, da dann bei Ausfall eines Rührwerks der Betrieb des Fermenters aufrechterhalten werden kann. Besonders bevorzugt werden drei Rührwerke eingesetzt, die auf einem gleichseitigen Dreieck angeordnet sind. Es können aber auch vier, fünf oder mehr Rührwerke eingesetzt werden, die linear, auf einem Quadrat, Pentagon oder entsprechendem Polygon angeordnet sind.

Vorzugsweise ist die Rührwerksanordnung so ausgestaltet, dass eine Anzahl von Rührpaddeln vorgesehen ist, die an der Rührwelle der jeweiligen Rührwerke vorzugsweise in unterschiedlichen Höhen angebracht sind und die vorzugsweise wenigstens teilweise verstellbar, insbesondere um eine von der Rührwelle weg weisenden Achse schwenkbar, sind. Mit einer solchen Rührpaddelanordnung ist eine Strömung innerhalb des Fermenters besonders gut steuerbar. Die Lage der Lageraufnahme des Rührwerksgestells und somit die Position der Rührwerksnaben ist vorzugsweise so gewählt, dass die Rührwerkspaddel etwa 1,5 m von der Behälterwand entfernt sind, um frisches Substrat sicher einzumischen.

In einer bevorzugten Ausführungsform ist die Rührwerksanordnung so dimensioniert, dass ein Volumen einer Rührwerkskaverne 5% bis 15%, vorzugsweise 8% bis 12%, besonders bevorzugt 9% bis 11% des Netto-Fermentervolumens beträgt. Als Volumen einer Rührwerkskaverne (nachstehend auch als Kavernengröße bezeichnet) wird im Sinne der Erfindung das von einem installierten Rührwerk überstrichene Volumen, verstanden. Es hat sich herausgestellt, dass eine gute Durchmischung bei geringem Aufwand an Rührenergie erreicht wird, wenn die Kavernengröße der aktiven Rührwerke in etwa 5% bis 7% des Behältervolumens und die Kavernengröße der installierten Rührwerke etwa 8% bis 12% beträgt, wobei als Kaverne (Rührwerkskaverne) in diesem Fall nur der vom Rührwerk überstrichende Zylinder definiert ist.

In einer bevorzugten Ausführungsform ist die Rührwerksanordnung so ausgestaltet, dass ein wenigstens Radialkräfte aufnehmendes oberes Rührwellenlager an dem Rührwerksgestell aufgenommen ist und ein wenigstens Radialkräfte aufnehmendes und vorzugsweise auch Axialkräfte aufnehmendes unteres Rührwellenlager vorgesehen ist, das zur Anbringung an einer Bodenplatte des Fermenterkörpers ausgebildet ist. Bei dieser Ausführungsform kann einerseits ein konstruktiver und materieller Aufwand vermindert werden, da das Rührwerksgestell nur im oberen Bereich eine Halte- und Positionierungsfunktion erfüllen muss und untere Lagerkräfte direkt in die Bodenplatte geleitet werden, andererseits können bei Nach- bzw. Umrüstung von bestehenden Fermentern bereits vorhandene Bodenlager verwendet werden.

In einer bevorzugten Ausführungsform ist der bzw. sind die Rührantrieb/e als Elektromotor/-en oder als Hydromotor/-en ausgebildet.

In einer bevorzugten Ausführungsform befindet sich das Getriebe im Rührantrieb. Dadurch ist ein langsames Drehen und somit eine laminare Umwälzung möglich.

In einer bevorzugten Weiterbildung ist vorgesehen, dass der/die Rührantrieb/-e mit einer Abdeckung abgedeckt ist, die gegen die Antriebsaufnahme statisch abgedichtet ist. Die Abdeckung ist vorzugsweise korrosionsfest ausgebildet und kann beispielsweise aus Edelstahl oder Kunststoff hergestellt sein. Der Rührwerksantrieb (Motor) ist in der Gasphase unterhalb des Fermenterdachs angeordnet. Die Atmosphäre in einem Biogasreaktor ist korrosiv. Sie enthält unter anderem Schwefelwasserstoff und geringe Mengen an Sauerstoff, die in Biofilmen zu Schwefelsäure reagieren kann. Der sich ausbildende Biofilm an Oberflächen in der Gasphase wirkt dabei nach kurzer Zeit wie eine Isolierung und verhindert eine Kühlung des Motors. Durch die erfindungsgemäße Abdeckung werden Schwefelsäurekorrosion und Biofilmbewuchs vom Motorgehäuse abgewehrt und daher auch die Kühlung des Motors verbessert. Der Motor kann auch aktiv gekühlt werden. Bei elektrischen Motoren wird zudem die Zünd-Gefahr von Biogas-Luft-Gemischen, wie sie häufig bei der Inbetriebnahme von Biogasanlagen auftreten, auf diese Weise umgangen.

Ein noch weiterer Gesichtspunkt der vorliegenden Erfindung betrifft einen Fermenter zur Herstellung von Biogas, mit den Merkmalen des Anspruchs 10.

Da der erfindungsgemäße Fermenter die erfindungsgemäße Rührwerksanordnung aufweist, werden die im Zusammenhang mit der Rührwerksanordnung beschriebenen Vorteile auch durch den Fermenter verwirklicht.

In einer bevorzugten Ausführungsform ist das Dach des Fermenterkörpers als ein Foliendach ausgebildet. Bei einem Foliendach kommen die Vorteile der Erfindung besonders gut zur Ausprägung, wie bereits im Zusammenhang mit dem Rührwerksgestell vorstehend beschrieben.

In einer bevorzugten Weiterbildung ist/sind der/die Rührantrieb/-e in einem fluidgeführten, vorzugsweise wassergeführten, System eingehüllt, wobei eine Ableitung des fluidführenden Systems aus dem Fermenterkörper vorgesehen ist. Mit anderen Worten, ein Zwischenraum zwischen einer oben beschriebenen Abdeckung ist mit Wasser oder einer anderen Flüssigkeit beaufschlagt. Dabei können die fluidgeführten Systeme mehrerer Rührantriebe untereinander durch eine Leitung innerhalb oder außerhalb des Fermenters verbunden sein. Die Fluidleitung wird vorzugsweise an mindestens zwei Punkten aus dem Behälter geführt. Dabei ist es auch möglich, Vor- und Rücklauf als Rohr-in-Rohr System zu realisieren, so dass nur eine Durchführung an der Fermenterwand notwendig ist. Der Fluidkreislauf kann in einen außenliegenden Fluidbehälter führen, unter dem sich die Umwälzpumpe für das Fluid befindet. Eine Temperaturüberwachung des Wassers kann einen drohenden Motorschaden anzeigen.

Wenn der/die Rührantrieb/-e aus einem Hydroantrieb/-en gebildet ist, ist es vorteilhaft, wenn auch die Hydraulikleitungen zu den Hydroantrieben in dem fluidgeführten System eingehüllt sind. Mit anderen Worten: bei der Ausführung der Rührwerksmotoren als Hydraulikmotoren werden die Hydraulikleitungen in die fluidführenden Leitungen gelegt. Sollte es zu einem Platzen der Hydraulikleitungen kommen, entweicht das giftige Hydrauliköl in den Fluidkreislauf, sodass eine Belastung des Behälterinhalts verhindert werden kann. Der Fluidbehälter ist dabei vorzugsweise so dimensioniert und ausgeführt, dass er zusätzlich zum Fluid auch die vollständige Menge Hydrauliköl aufnehmen kann. Zusätzlich kann das Fluid in regelmäßigen Abständen auf Kontamination durch Hydrauliköl überwacht werden. Die Überwachung kann automatisch (z.B. Trübung, Leitfähigkeit) oder durch Inaugenscheinnahme durch den Betreiber erfolgen. Das Fluidsystem kann zusätzlich auf die optimale Betriebstemperatur der Hydraulikanlage temperiert werden. Ein Motorschaden kann dann durch eine Reduzierung der benötigten Heizleistung bzw. Erhöhung der Kühlleistung detektiert werden. Anders als bei den Hydraulikmotoren ist es bei den elektrischen Motoren nicht notwendig, die elektrischen Leitungen durch die Fluidleitungen zu ziehen.

Ein noch weiterer Gesichtspunkt der vorliegenden Erfindung betrifft eine Biogasanlage, welche wenigstens einen Fermenter aufweist, der nach der vorstehenden Beschreibung des vorherigen Erfindungsgesichtspunkts ausgebildet ist.

Da die erfindungsgemäße Biogasanlage den erfindungsgemäßen Fermenter aufweist, werden die im Zusammenhang mit dem Fermenter beschriebenen Vorteile auch durch die Biogasanlage verwirklicht.

In einer bevorzugten Ausführungsform weist die Biogasanlage wenigstens einen Fermenter einer ersten Fermentierungsstufe und wenigstens einen Fermenter einer zweiten Fermentierungsstufe auf, wobei wenigstens einer des wenigstens einen Fermenters der ersten Fermentierungsstufe und wenigstens einer des wenigstens einen Fermenters der zweiten Fermentierungsstufe nach der vorstehenden Beschreibung des vorherigen Erfindungsgesichtspunkts ausgebildet ist. Unter einer ersten Fermentierungsstufe wird im Sinne der Erfindung eine Fermentierungsstufe verstanden, die hauptsächlich mit frischem Substrat beschickt wird, und unter einer zweiten Fermentierungsstufe wird eine Fermentierungsstufe verstanden, die hauptsächlich mit Gärsubstrat, das bereits die erste Fermentierungsstufe durchlaufen hat, beschickt wird. Die erste Fermentierungsstufe kann als eine hauptsächliche oder primäre Fermentierung verstanden werden, und ein Fermenter der ersten Fermentierungsstufe wird im Rahmen dieser Anmeldung auch als ein Hauptfermenter bezeichnet. Die zweite Fermentierungsstufe kann als eine nachrangige oder sekundäre Fermentierung verstanden werden, und ein Fermenter der zweiten Fermentierungsstufe wird im Rahmen dieser Anmeldung auch als ein Nachgärer bezeichnet. Der Nachgärer kann auch eine Funktion eines Gasspeichers aufweisen und kann gasseitig mit dem Hauptfermenter verbunden sein.

In einer bevorzugten Ausführungsform weist die Biogasanlage wenigstens ein Blockheizkraftwerk auf, welches mit Biogas, welches durch die Biogasanlage erzeugt wird, betreibbar ist. Das durch die Biogasanlage erzeugte Biogas kann auf diese Weise direkt verwertet werden, indem in dem Blockheizkraftwerk elektrische Energie und Wärme erzeugt werden. Die erzeugte elektrische Energie kann in ein öffentliches Stromnetz gespeist oder direkt für den Betrieb der Anlage oder Nebenanlagen wie etwa eines zugehörigen landwirtschaftlichen Betriebs genutzt werden. Die erzeugte Wärme kann in ein öffentliches Fernwärmenetz gespeist oder direkt für den Betrieb der Anlage oder Nebenanlagen wie etwa eines zugehörigen landwirtschaftlichen Betriebs genutzt werden. Im Übrigen kann selbstverständlich das erzeugte Biogas alternativ oder optional vollständig oder teilweise (soweit nicht im Blockheizkraftwerk genutzt) auch in ein öffentliches Gasversorgungsnetz gespeist werden.

In einer bevorzugten Ausführungsform weist die Biogasanlage eine Biogasaufbereitungseinrichtung auf. Dadurch kann das erzeugte Biogas so aufbereitet werden, dass es für eine vorgesehene energetische oder stoffliche Nutzung geeignet ist. Die Aufbereitung kann ausgewählte oder alle der folgenden Schritte umfassen: Entschwefelung, Trocknung, Kohlendioxidabtrennung, Konditionierung. Eine Konditionierung ist insbesondere bei Einspeisung in ein öffentliches Gasversorgungsnetz vorteilhaft, um die vereinbarte Qualität und Beschaffenheit beispielsweise auf Erdgasstandard sicherzustellen.

In einer weiteren bevorzugten Ausführungsform ist eine Gärrestseparationseinheit vorgesehen, welche ausgebildet ist, um Fermentationsrückstände und Reststoffe des Betriebs in den oder die Fermenter der Biogasanlage zu rezirkulieren und/oder gesondert bereitzustellen. Durch Rezirkulierung kann der Flüssigkeits- bzw. Feststoffgehalt des Fermenters gesteuert werden. Durch gesonderte Bereitstellung können Reststoffe beispielsweise als Festdünger und/oder Flüssigdünger vertrieben oder direkt für den Betrieb etwa eines zugehörigen landwirtschaftlichen Betriebs genutzt werden.

Ein noch weiterer Gesichtspunkt der vorliegenden Erfindung betrifft ein Verfahren zur Erzeugung von Biogas durch Fermentation eines Substrats in einem Fermenter, der nach der vorstehenden Beschreibung eines vorherigen Erfindungsgesichtspunkts ausgebildet ist, wobei das Substrat in dem Fermenter durch die Rührwerksanordnung wenigstens teilweise laminar bzw. im Wesentlichen laminar durchmischt wird.

Da das erfindungsgemäße Verfahren mit Hilfe des erfindungsgemäßen Fermenters durchgeführt wird, werden die im Zusammenhang mit dem Fermenter beschriebenen Vorteile, insbesondere im Hinblick auf die Verwendung dickflüssigeren Substrats, auch durch das Verfahren verwirklicht.

In einer bevorzugten Ausführungsform werden als Bestandteile des Substrats landwirtschaftliche Produkte, insbesondere Energiepflanzen und deren Früchte und/oder Silagen, Gülle und/oder Mist eingesetzt. Mit diesen Bestandteilen, die regelmäßig beim Betrieb eines landwirtschaftlichen Betriebs anfallen oder auch gezielt erzeugbar sind, lässt sich eine Biogasanlage besonders wirtschaftlich betreiben. Darüber hinaus kann auch Biomüll als Substrat eingesetzt werden. Die Vorteile der vorliegenden Erfindung kommen besonders deutlich zum Tragen, da die genannten Substratbestandteile für eine laminare Durchmischung besonders gut geeignet sind.

In einer bevorzugten Ausführungsform wird aus einem fluidführenden, insbesondere wasserführenden, System, welches den Rührantrieb bzw. die Rührantriebe umhüllt, aus dem Fermenter ausgeleitetes Fluid hinsichtlich seiner Temperatur überwacht, um einen Rührwerksschaden zu detektieren. Ein Schaden am Rührwerk (insbesondere Motorschaden, zugesetzte Lager) führt oft zu einer erhöhten Leistungsaufnahme am Rührwerksantrieb, die wiederum zu einer erhöhten Wärmeerzeugung führt. Diese kann durch eine Temperaturüberwachung des umhüllenden Fluids gut detektiert werden. So kann eine Betriebssicherheit der Anlage weiter verbessert werden.

In einer bevorzugten Ausführungsform wird aus einem fluidführenden, insbesondere wasserführenden, System, welches den Rührantrieb bzw. die Rührantriebe umhüllt, aus dem Fermenter ausgeleitetes Wasser auf Verunreinigungen, insbesondere durch Hydrauliköl, untersucht. So kann eine Leckage am Rührantrieb (bspw. Hydromotor) rasch erkannt und eine Betriebssicherheit der Anlage weiter verbessert werden. Die Überwachung kann automatisch (z.B. Trübung, Leitfähigkeit) oder durch Inaugenscheinnahme durch den Betreiber erfolgen.

In einer bevorzugten Ausführungsform wird ein fluidführendes, insbesondere wasserführendes, System, welches den/die Rührantrieb/-e umhüllt, hinsichtlich seiner Temperatur geregelt. So kann auch ein Elektromotor optimal gekühlt werden oder eine optimale Betriebstemperatur von für ein, in einem Hydroantrieb verwendetem Hydrauliköl, erreicht werden. Auf diese Weise kann die Betriebssicherheit und Wirtschaftlichkeit der Anlage weiter verbessert werden.

In einer bevorzugten Ausführungsform wird der wenigstens eine Fermenter so betrieben, dass mehrere Rührwerke zyklisch derart betrieben werden, dass stets wenigstens ein Rührwerk der mehreren Rührwerke im Wechsel stillsteht. Auf diese Weise kann eine besonders günstige Umschichtung erzielt werden, was die Effizienz der Anlage weiter verbessern kann. Vorteilhaft kann ein Umschaltzeitpunkt der betriebenen bzw. stillstehenden Rührwerke auf einen Zeitpunkt abgestimmt werden, an welchem Rührwerkskavernen benachbarter Rührwerke sich zu einer Rührwerkskavernen verbunden haben. Erfahrungsgemäß hat sich ein Umschaltintervall von zwischen 5 und 30 Minuten, vorzugsweise zwischen 8 und 20 Minuten, für eine bevorzugte Substratmischung von 6 Gewichtsanteilen Mais, 8,5 Gewichtsanteilen Gras und 3 Gewichtsanteilen Wasser insbesondere zwischen 10 und 15 Minuten als besonders geeignet herausgestellt.

In einer bevorzugten Ausführungsform wird das erzeugte Biogas einem Brenner, Blockheizkraftwerk und/oder einer Biogasaufbereitungseinrichtung zugeführt.

Die vorliegende Offenbarung betrifft ein Verfahren zum Umrüsten eines Fermenters zur Erzeugung von Biogas, mit den Schritten:

Einbau eines Rührwerksgestells, das nach der vorstehenden Beschreibung eines vorherigen Erfindungsgesichtspunkts ausgebildet ist, in einen Fermenterkörper.

Da das Verfahren das erfindungsgemäße Rührwerksgestell verwendet, werden die im Zusammenhang mit dem Rührwerksgestell beschriebenen Vorteile auch durch das Verfahren verwirklicht.

Weitere Verfahrensschritte können umfassen

- Demontage von vorhandenen Rührantrieben,
- Demontage von vorhandenen Rührwellen,
- Demontage von vorhandenen oberen Rührwellenlagern,
- Demontage eines Fermenterdachs,
- Demontage von vorhandenen unteren Rührwellenlagern, wenn die Umrüstung den Einbau neuer unterer Rührwellenlager vorsieht,
- Montage von unteren Rührwellenlagern am Fermenterboden oder am Rührwerksgestell,
- Montage von oberen Rührwellenlagern am Rührwerksgestell,
- Einbau von Rührwellen in den vorgesehenen Rührwellenlagern,
- Montage von Rührantrieben am Rührwerksgestell,
- Anschluss von Versorgungsleitungen,
- Abdichten von Durchlässen der Versorgungsleitungen in der Fermenterwand,
- Montage eines neuen oder des alten Fermenterdachs,
- Schließen von Durchlässen des Fermenterdachs, wenn das alte Fermenterdach wiederverwendet wird,
- Einbringen von Durchlässen in der Fermenterwandung.

Die Erfindung wird nachstehend anhand der beigefügten Zeichnungen beispielhaft genauer erläutert. Dabei zeigt

Fig. 1       eine räumliche Darstellung eines Fermenters mit einem Rührwerksgestell und drei Rührwerken als ein Ausführungsbeispiel der vorliegenden Erfindung;

Fig. 2    eine Einzelheit "II" des Fermenters von Fig. 1;

Fig. 3    eine Einzelheit "III" der Darstellung des Rührwerks in Fig. 2;

Fig. 4    eine Draufsicht des Fermenters von Fig. 1 in einem Schnitt entlang einer Linie IV in Blickrichtung des angegebenen Dreiecks;

Fig. 5    eine Querschnittsansicht des Fermenters von Fig. 1 in einem Schnitt entlang einer Linie V in Fig. 4 in Blickrichtung des angegebenen Dreiecks;

Fig. 6    eine schematische Draufsicht einer Biogasanlage mit zwei Fermentern als ein weiteres Ausführungsbeispiel der vorliegenden Erfindung;

Fig. 7    ein Blockschaubild der Biogasanlage von Fig. 6 in einer schematisierten Seitenansicht;

Fig. 8    ein Diagramm eines Verlaufs einer Schubspannung über die Scherrate für verschiedene Substratmischungen;

Fig. 9    ein Diagramm eines Verlaufs einer scheinbaren Viskosität über die Scherrate für die gleichen Substratmischungen;

Fig. 10a, 10b    jeweils eine räumliche Darstellung eines Rührwerksgestells nach einem weiteren Ausführungsbeispiel.

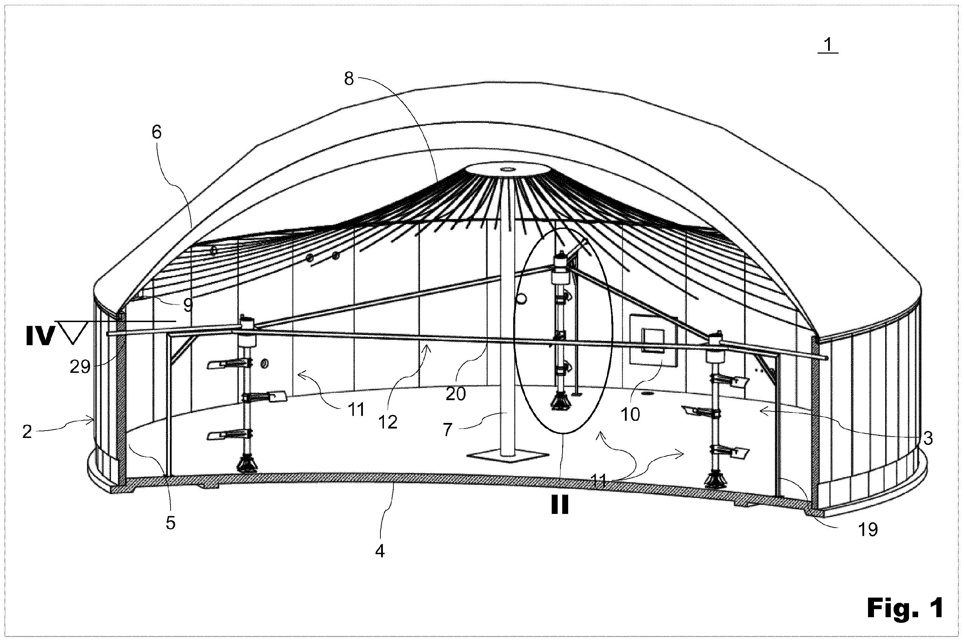

Der Aufbau eines Fermenters 1 gemäß einem ersten Ausführungsbeispiel der vorliegenden Erfindung wird nun anhand der Darstellung in Fign. 1 bis 5 erläutert.

Gemäß der Darstellung in Fig. 1, die eine perspektivische Darstellung des aufgeschnittenen Fermenters 1 zeigt, weist der Fermenter 1 einen Fermenterkörper 2, der einen Innenraum des Fermenters 1 definiert, und eine Rührwerksanordnung 3 auf. Der Fermenterkörper 2 weist eine Bodenplatte 4, eine Seitenwandung 5 und ein Dach 6, das hier ein doppelwandiges Foliendach ist, auf. Eine Stützsäule 7 erhebt sich von der Mitte der Bodenplatte 4 bis knapp unter das Dach 6, wo sie in einer verbreiterten Ablage (nicht näher bezeichnet) endet. Vom Außenrand der Ablage erstreckt sich eine Abspannung 8, die aus mehreren leicht durchhängenden Seilen besteht, zum oberen Rand der Seitenwandung 5. Da der Fermenter 1 im normalen Betrieb unter einem leichten Überdruck steht, ist das Dach 6 normalerweise aufgebläht. Bei Nachlassen des Überdrucks kann das Dach 6 aber zusammensinken. Dann kann sich das Dach 6 auf der Ablage der Stützsäule 7 und der Abspannung 8 ablegen. Im oberen Bereich der Seitenwandung 5 ist eine Zuführöffnung 9 zum Zuführen von Substrat vorgesehen und im unteren Bereich der Seitenwandung 5, in Umfangsrichtung von der Zuführöffnung 9 entfernt, ist eine Entnahmeöffnung 10 zur Entnahme von Gärsubstrat vorgesehen. Es sind weitere nicht näher bezeichnete Öffnungen in der Seitenwandung 5 für verschiedene Zwecke (Leitungsführungen etc.) ausgebildet. Alle Öffnungen sind während des Betriebs des Fermenters 1 abgedichtet.

Im Inneren des Fermenterkörpers 2 ist unterhalb der Abspannung 8 die Rührwerksanordnung 3 angeordnet. Die Rührwerksanordnung 3 weist im vorliegenden Ausführungsbeispiel drei Rührwerke 11 auf, die von einem Rührwerksgestell 12 gehalten werden.

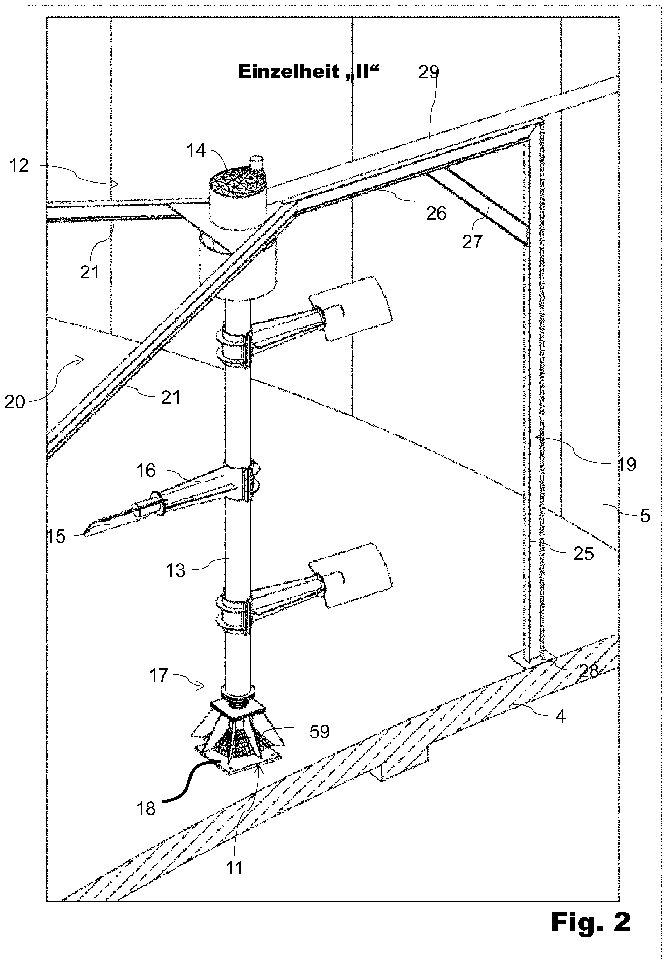

In Fig. 2 ist eine Einzelheit "II" aus Fig. 1 vergrößert dargestellt. Demnach weist ein Rührwerk 11 eine Rührwelle 13 auf, die von einem Rührantrieb 14 angetrieben wird. Drei Rührpaddel 15 sind jeweils mittels einer Paddelstange 16 in unterschiedlichen Höhen an der Rührwelle 13 befestigt. Im oberen Bereich ist die Rührwelle 13 an dem Rührwerksgestell 12 und im unteren Bereich in einem unteren Rührwellenlager 17 gelagert. Das untere Rührwellenlager 17 ist, wie es in der deutschen Gebrauchsmusteranmeldung DE 20 2013 104 292.7 beschrieben ist, ausgebildet. Am unteren Ende der Rührwelle 13 ist ein nach unten offener Findungstrichter 59 angeordnet, der drehbar gegenüber der Rührwelle gelagert ist. An einer Basisplatte 18 ist ein Zentrierstummel (nicht dargestellt) vertikal angeordnet. Die Basisplatte ist an der Bodenplatte 4 des Fermenterkörpers befestigt.

Das Rührwellenlager kann auch einen Aufbau aufweisen, wie er in der internationalen Anmeldung PCT/EP2006/008488 der Anmelderin, die als WO 2007/025739 A2 veröffentlicht ist und auf deren Inhalt insoweit verwiesen wird, im Einzelnen beschrieben ist. Das untere Rührwellenlager 17 vermittelt der Rührwelle 13 eine axiale und radiale Führung.

Bei beiden oben erläuterten unteren Rührwellenlagern ist die Rührwelle lösbar mit einem an der Bodenplatte befestigten Basisplatte verbunden, wobei die Rührwelle aus der Basisplatte durch eine Bewegung vertikal nach oben gelöst werden kann. In Verbindung mit dem Rührwerksgestell erlaubt ein solches unteres Rührwellenlager eine im Fermenter frei stehende Rührwelle. Das einer Rührwelle zugeordnete Rührwerksgestell kann auch mit der Bodenplatte und/oder der Seitenwandung und/oder mit einem weiteren Rührwerksgestell verbunden sein, um ein oberes Rührwellenlager zu halten.

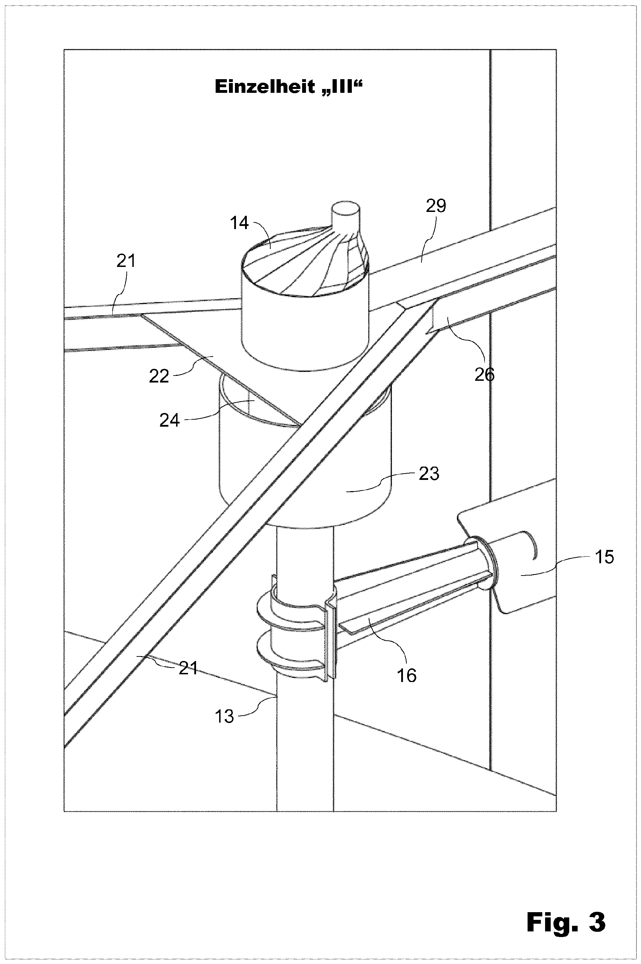

Das Rührwerksgestell 12 weist gemäß der Darstellung in Fig. 1 einen zentralen Rahmen 20 und drei seitliche Verankerungen 19 auf. Wie in Fig. 1 und in Fig. 2, die eine Einzelheit "II" in Fig. 1 vergrößert wiedergibt, gesehen, weist der Rahmen 20 drei Rahmenstreben 21 auf, die zusammen ein Dreieck bilden, wobei die Enden der Rahmenstreben 21 durch ein Knotenblech 22 jeweils verbunden sind. Wie in Fig. 3, die eine Einzelheit "III" in Fig. 2 noch weiter vergrößert wiedergibt, bildet das Knotenblech 22 eine Halterung bzw. Aufnahme für den Rührantrieb 14. Unterhalb des Knotenblechs 22 ist eine manschettenförmige Lageraufnahme 23 befestigt. Die Lageraufnahme 23 nimmt ein oberes Rührwellenlager 24 auf, das die Rührwelle 13 radial abstützt. Mit anderen Worten, der Rührantrieb 14 und die Rührwelle 13 erstrecken sich vertikal im Bereich der Verbindungsstelle zweier Rahmenstreben 21, wobei die gemeinsame Achse durch das Knotenblech 22 innerhalb der von den Rahmenstreben 21 aufgespannten Ecke verläuft. Jeder Ecke des Rahmens 20 ist ein Rührwerk 11 (Rührantrieb 14, Rührwelle 13) zugeordnet. Wie am besten in Fig. 2 zu sehen, weist die Verankerung 19 eine Vertikalstrebe 25 und eine Horizontalstrebe 26 auf, die auf Gehrung rechtwinklig aneinanderstoßen und in der gemeinsamen Ecke durch eine Winkelstrebe 27 versteift sind, sodass die Verankerung 19 eine Art Galgenform aufweist. Die Vertikalstrebe 25 weist am freien (unteren) Ende eine Ankerplatte 28 auf, die an der Bodenplatte 4 des Fermenterkörpers 2 befestigt ist. Die Horizontalstrebe 26 ist an einer Ecke, an welcher die zwei Rahmenstreben 21 aneinanderstoßen, mit diesen verbunden.

Zusammenfassend bilden die drei Rahmenstreben 21 den Rahmen 20 in Form eines gleichseitigen Dreiecks, wobei die Ecken jeweils durch ein jeweiliges Knotenblech 22 versteift sind, und drei Verankerungen 19 vorgesehen sind, welche jeweils an einer Ecke des Rahmens 20 angreifen und den Rahmen 20 mit der Bodenplatte 4 verbinden. Somit wird der Rahmen 20 durch die Verankerungen 19 relativ zu dem Fermenterkörper 2 verankert, wobei der Rahmen 20 die Antriebe 14 und oberen Rührwellenlager 24 aufnimmt, und wobei die gesamte Rührwerksanordnung 3 innerhalb des Behälterkörpers 2 aufgenommen ist.

Alle Bestandteile des Rührwerksgestells 12, mithin die Rahmenstreben 21, die Knotenbleche 22, die Horizontalstreben 26, die Vertikalstreben 25, die Winkelstreben 27 und die Ankerplatte 28 sind aus Stahl hergestellt und sind, wo sie aneinander stoßen, miteinander verschweißt. Die Streben 21, 25, 26, 27 weisen jeweils ein I-Profil auf.

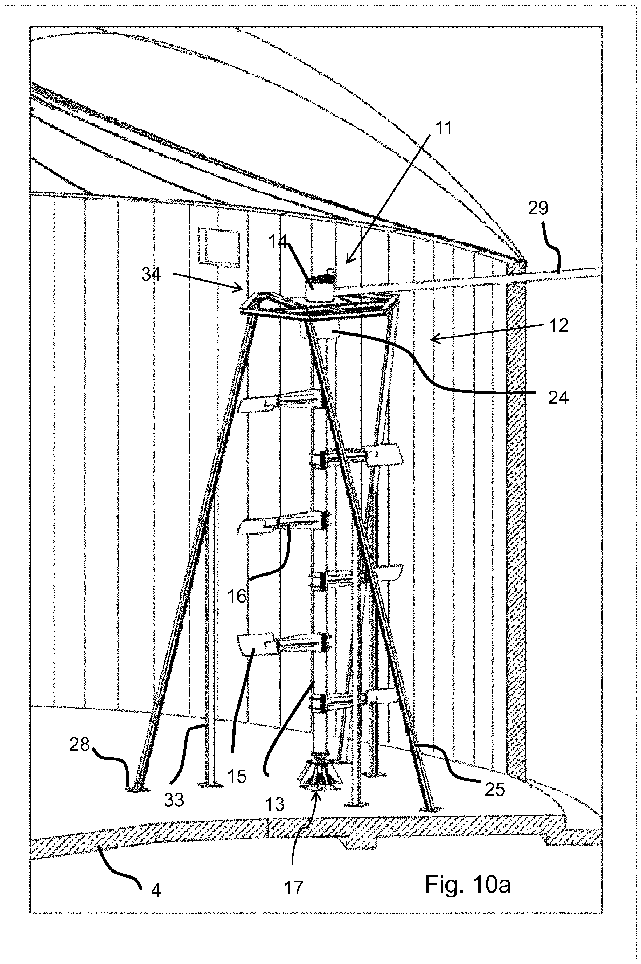

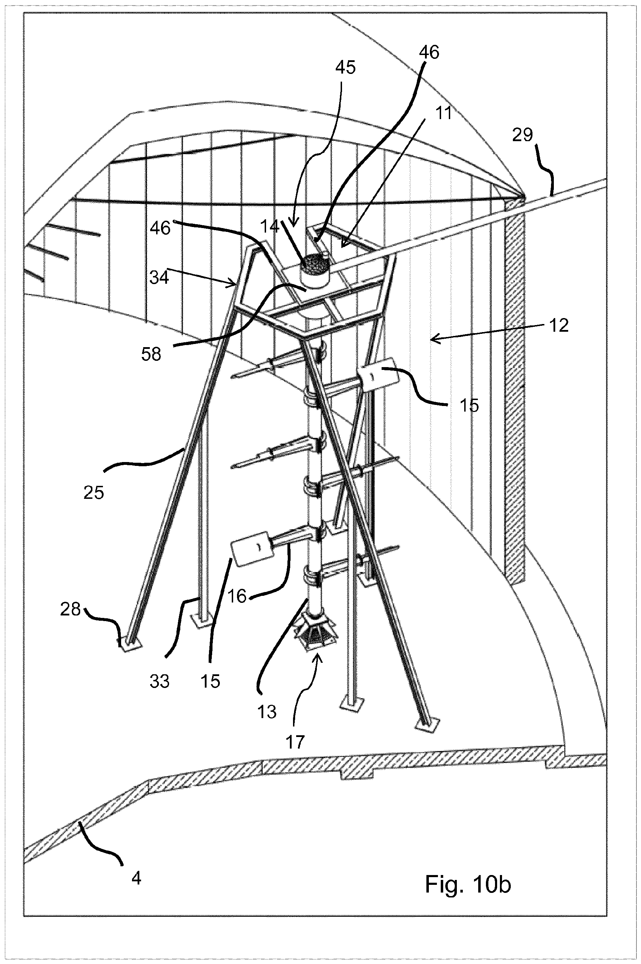

In Figuren 10a und 10b ist ein weiteres Ausführungsbeispiel eines Rührwerksgestells 12 für den Fermenter 1 gezeigt. Gleiche Teile sind mit gleichen Bezugszeichen wie im oben erläuterten Ausführungsbeispiel versehen und werden nicht nochmals im Detail erläutert. Dieses Rührwerksgestell 12 ist zum Halten eines Rührwerks 11 mit einer Rührwelle 13, einem Rührantrieb 14, Rührpaddeln 15, Paddelstangen 16, einem oberen Rührwellenlager 24 und einem unteren Rührwellenlager 17 ausgebildet.

Das untere Rührwellenlager ist genauso wie beim obigen Ausführungsbeispiel als lösbares Rührwellenlager ausgebildet. Es kann der Ausführungsform nach dem deutschen Gebrauchsmuster DE 20 2013 104 292.7 oder der Ausführungsform nach der WO 2007/025739 A2 entsprechen.

Im Unterschied zum oben erläuterten Ausführungsbeispiel ist dieses Rührwerksgestell 12 lediglich zum Halten einer einzigen Rührwelle 13 bzw. eines einzigen Rührwerkes 11 vorgesehen. In einem Fermenter können mehrere dieser Rührwerksgestelle 12 mit jeweils einem Rührwerk 11 angeordnet sein.

Das Rührwerksgestell 12 weist drei Vertikalstreben 25 auf, die gegenüber der Bodenplatte 4 schräg angeordnet sind. Die Vertikalstreben 25 werden durch Stützstreben 33 gestützt, die etwa auf halber Höhe der Vertikalstreben 25 mit diesen verbunden sind und sich von der Verbindungsstelle senkrecht nach unten bis zur Bodenplatte 4 erstrecken. Sowohl die Vertikalstreben 25 als auch die Stützstreben 33 weisen an ihrem unteren Ende jeweils eine Ankerplatte 28 auf. Die Vertikalstreben 25 enden mit ihrem oberen Ende an einem Gerüstkranz 34, der aus mehreren Streben zusammengesetzt ist. Der Gerüstkranz 34 weist einen Aufnahmebereich 45 auf, der zu einer Seite offen ist. Im vorliegenden Ausführungsbeispiel ist der Aufnahmebereich durch zwei parallele Streben 46 begrenzt.

Das Rührwerk 11 weist an seinem oberen Endbereich ein Kopplungselement 58 auf, das im vorliegenden Ausführungsbeispiel als Platte ausgebildet ist. Diese Platte liegt auf dem Gerüstkranz 34 auf und weist nach unten vorstehende Vorsprünge (nicht dargestellt) auf, welche formschlüssig an den Streben 46 des Gerüstkranzes 34 anliegen. Hierdurch ist die Lage des Kopplungselementes 58 bezüglich des Rührwerksgestells 12 eindeutig festgelegt. An dem Kopplungselement 58 ist das obere Rührwellenlager 24 sowie der Rührantrieb 14 befestigt. Im Übrigen sind das obere Rührwellenlager 24 und der Rührantrieb 14 genauso wie beim oben erläuterten Ausführungsbeispiel ausgebildet. Vom Rührantrieb 14 führt radial nach außen eine Zuleitung 29, die unten näher erläutert wird.

Zum Austauschen eines Rührwerkes kann das Dach 6 bereichsweise vom Fermenterkörper 2 gelöst und gefaltet werden, sodass das Rührwerksgestell 12 freiliegt. Dann kann mit einem Kran oder einem Lader mit Teleskopausleger das obere Ende des Rührwerkes 11 an der entsprechenden Hubeinrichtung eingehängt werden. Nach dem Lösen der Zuleitung 29 kann das Rührwerk angehoben werden, wobei es beim Anheben so zu drehen ist, dass die Paddel zusammen mit den Paddelstangen 16 immer entlang des Aufnahmebereichs 45 angeordnet sind. Hierdurch ist es möglich, in Verbindung mit einem lösbaren unteren Rührwellenlager 17 ein Rührwerk 11 an einem mit Biomasse gefüllten Fermenter 1 auszutauschen. Der seitlich offene Aufnahmebereich des Gerüstkranzes 34 erlaubt somit das Entnehmen des Rührwerks 11, wobei der Betrieb des Fermenters nur kurzzeitig unterbrochen werden muss und ein Entleeren des Fermenters nicht notwendig ist.

Das Anheben des Rührwerkes 11 kann auch derart erfolgen, dass das Rührwerk 11 zunächst ein Stück angehoben wird, bis die Verbindung am unteren Rührwellenlager 17 gelöst ist. Dann kann das Rührwerk 11 seitlich aus dem Aufnahmebereich 45 herausgeschwenkt werden. Wenn das vorstehende Rührwerk 11 außerhalb des Rührwerksgestells 12 angeordnet ist, kann es vollständig angehoben und aus dem Fermenter entfernt werden.

Beim oben erläuterten Ausführungsbeispiel mit einem Rahmen 20, der sich zwischen mehreren Aufnahmen für Rührwerke 11 erstreckt, kann es auch zweckmäßig sein, einen seitlich offenen Aufnahmebereich, insbesondere am Knotenblech 22, vorzusehen, sodass eine Rührwelle nach oben angehoben werden kann.

Das Einsetzen eines Rührwerks 11 erfolgt in umgekehrter Weise.

In den vorliegenden Ausführungsbeispielen sind die Rührantriebe 14 Hydroantriebe. Je eine Zuleitung 29 erstreckt sich zur Versorgung der Rührantriebe 14 von den jeweiligen Rührantrieben 14 radial nach außen, wobei sie auf der Horizontalstrebe 26 der jeweiligen Verankerung 19 aufliegt, und führt dann durch eine Öffnung (nicht näher bezeichnet) in der Seitenwandung 5 nach außerhalb des Fermenterkörpers 2. Obschon nicht näher dargestellt, weisen die Rührantriebe 14 jeweils eine Umhüllung auf, die mit dem jeweiligen Kontenblech 22 dicht abschließt. Ferner sind die Zuleitungen 29 Kombinationsleitungen, in welchen Vor- und Rücklauf eines Hydraulikkreises in einem Mantelrohr verlaufen. Die Umhüllung des Rührantriebs 14 und das Mantelrohr der Zuleitung 29 sind fluidkommunizierend verbunden und bilden ein wassergeführtes System, das außerhalb des Fermenterkörpers 2 zu einem Wasserbehälter (nicht näher dargestellt) führt. Durch das wassergeführte System kann das hydraulische Antriebssystem gekühlt bzw. temperiert (d.h., ggf. auch erwärmt) werden, können Leckagen von Hydrauliköl aufgefangen und abgeführt werden und kann auch eine Überwachung auf Funktionsstörungen erfolgen.

Der Betrieb des Fermenters 1 wird nachfolgend anhand der Darstellung in Fign. 1, 4 und 5 näher erläutert, wobei letztere eine Draufsicht bzw. einen Querschnitt des kreisförmigen Fermenters 1 mit seinen drei Rührwerken 11 zeigen.

Die Inputstoffe werden oben am Fermenter 1 durch die Zuführöffnung 9 zugeführt. Geeignete Inputstoffe für die Trockenfermentation sind im Wesentlichen alle verwertbaren stapelfähigen Biomassen mit einem Trockenrückstand von zumindest 25 %. Geeignete Inputstoffe sind z.B. Silomais, Getreide-GPS, Grassilage, Zuckerrübensilage, Futterrübensilage und Getreide (Roggen, Triticale, Gerste, Weizen).

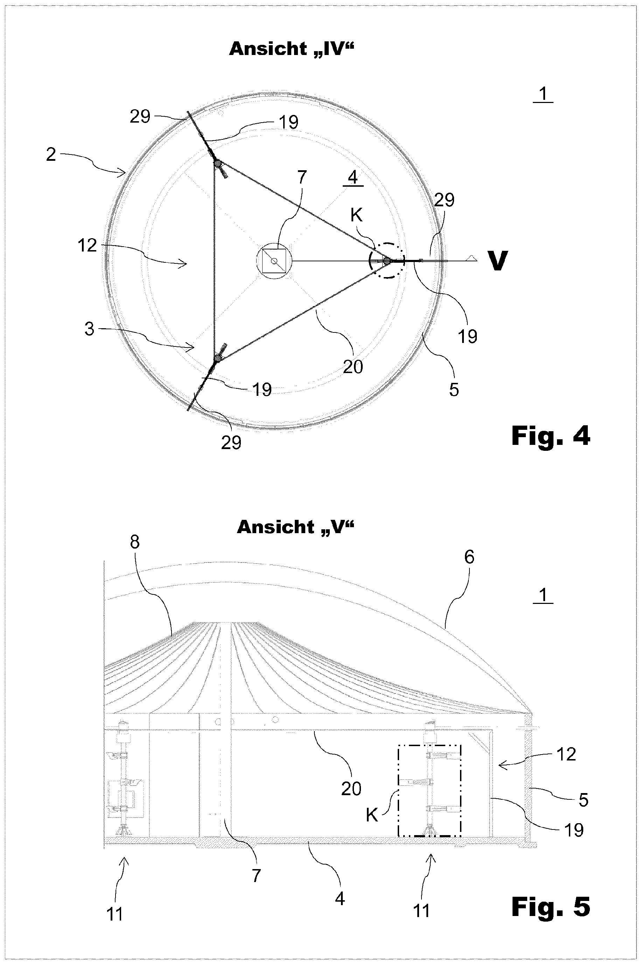

Die Rührwerke 11 werden in gleicher Drehrichtung betrieben. Die Drehgeschwindigkeit ist gering. Sie beträgt maximal 60 U/min. Typischerweise liegt die Drehgeschwindigkeit im Normalbetrieb im Bereich von 0 bis 20 U/min. Es hat sich gezeigt, dass bei dieser Anordnung mit vertikal ausgerichteter Rührwelle 13 und einer langsamen, kontinuierlichen Drehung des Rührwerkes 11 das gesamte Substrat des Fermenters 1 bewegt wird. Hierfür ist es vorteilhaft, wenn der Fermenter 1 in seiner Draufsicht (Fig. 4) kreisförmig ausgebildet ist.

Es hat sich zudem gezeigt, dass zum Bewegen des vollständigen Substrates lediglich ein einziges Rührwerk 11 notwendig ist. Aus Sicherheitsgründen sind vorzugsweise jedoch mindestens zwei, hier drei, Rührwerke eingebaut, um bei Ausfall eines Rührwerkes 11 das Substrat kontinuierlich in Bewegung halten zu können. Hierdurch wird ein Ansteigen des Flüssigkeitsspiegels im Fermenter 1 durch Biogaseinschlüsse in den Schwimmdecken verhindert.

Bei der kontinuierlichen rotatorischen Umwälzung des Substrates (nicht näher dargestellt) im Fermenter 1 stellen sich drei Abbauzonen ein, die im Fermenter 1 übereinander geschichtet sind. Die oberste Zone ist eine Verflüssigungszone, die mittlere Zone eine Methanisierungszone und die untere Zone eine Entnahmezone.

Diese drei Abbauzonen stellen sich ein, wenn die Rührpaddel 15 im Wesentlichen vertikal ausgerichtet sind, so dass im Substrat keine nennenswerte Bewegung nach oben oder unten erzeugt wird. Derart vertikal ausgerichtete Rührpaddel 15 sprechen das Substrat im Wesentlichen nur in einzelnen Ebenen an, so dass die Ebenen nicht durchmischt werden. Vorzugsweise werden die obersten Paddel 15 bzw. die sich in der Verflüssigungszone befindlichen Rührpaddel 15 gegenüber der Vertikalen etwas schräg gestellt, so dass neu hinzugefügtes Substrat unverzüglich mit dem Substrat der Verflüssigungszone vermischt wird.

In einer anderen Betriebsart werden die Rührpaddel 15 gegenüber der Vertikalen geneigt. Bei einem Neigungswinkel von 20° bis 70° und insbesondere von 30° bis 60° wird das Substrat im erheblichen Umfang vertikal durchmischt. Sind alle Rührpaddel 15 einer Rührwelle 13 in die gleiche Richtung geneigt, so entsteht eine vertikale Strömung des Substrates entlang der Rührwelle 13 über die gesamte Füllhöhe des Substrates. Je nach der Richtung der Rührwelle 13 ist die Strömung entlang der Rührwelle 13 nach oben oder nach unten gerichtet. In einem solchen Betrieb stellen sich keine horizontalen Abbauzonen ein, sondern das gesamte Substrat wird gleichmäßig vermischt.

In der Verflüssigungszone befindet sich das noch wenig abgebaute Substrat, das aufgrund des hohen Organikgehalts die geringste Dichte aufweist. Das Material sinkt mit fortschreitender Verflüssigung durch den biologischen Abbau aus der Verflüssigungszone in die Methanisierungszone ab. In der Methanisierungszone wird das meiste Methan freigesetzt. Stark abgebautes Substrat gelangt auf Grund der höheren Dichte in die Entnahmezone, aus welcher es durch die Entnahmeöffnung 10 (Fig. 1) abgeführt wird. Genauer gesagt ist an der Entnahmeöffnung 10 am unteren Randbereich der Seitenwandung 5 des Fermenters 1 eine Förderpumpe (nicht dargestellt) gekoppelt, die das vergorene Material (Gärsubstrat) aus dem Fermenter 1 entnimmt und zur weiteren Verarbeitung befördert. Da die Zuführöffnung 9 oben und die Entnahmeöffnung 10 unten am Fermenter angeordnet sind, wird der Fermenter 1 von oben nach unten beschickt. Vorzugsweise sind die Zuführöffnung 9 und die Entnahmeöffnung 10 diametral gegenüberliegend am Fermenter 1 angeordnet, so dass das Substrat beim Durchfluss durch den Fermenter diesen einmal vollständig queren muss.

Das hierbei entstandene Biogas sammelt sich unterhalb des Fermenterdachs 6 an und wird an einer Öffnung (nicht näher bezeichnet) im oberen Bereich der Seitenwandung 5 abgeführt.

Der Füllstand im Fermenter 1 wird mittels einer Radarsonde (nicht dargestellt) überwacht. Übersteigt der Füllpegel ein bestimmtes Niveau, schaltet sich automatisch die Förderpumpe für die Entnahme des abgebauten Materials ein.

Durch den Bau und Betrieb von verschiedenen laminar durchmischten Biogasanlagen für landwirtschaftliche Substrate ist den Erfindern bekannt, dass in diesem Einsatzfall das Scale-Up-Kriterium konstante Rührerspitzengeschwindigkeit angewendet werden kann. Das Gärsubstrat kann für den in Frage kommenden Anwendungsfall als ein Bingham-Fluid beschrieben werden, bei dem die Grenzschubspannung dominiert. Für den Fall, dass drei Rührwerke 11 in einem 8 m hohen Behälter eingesetzt werden sollen, sind Rührwerksdurchmesser, Drehzahl und Wellenleistung in der Tabelle 2 wiedergegeben.

Allgemein gilt bei laminarem Rühren der Zusammenhang

$$P = \eta \cdot n^2 \cdot d^3.$$

Für den Spezialfall einer Biogasanlage können nach Erkenntnissen der Erfinder sinnvolle Kriterien festgelegt und Vereinfachungen gemacht werden. So kann für die Viskosität eines Bingham-Fluids mit dominanter Grenzschubspannung und vernachlässigbarem scherratenabhängigen Schubspannungsanteil der Zusammenhang

$$\eta \sim \frac{1}{n}$$

angenommen werden.

Ein sinnvolles Scale-Up-Kriterium bei laminar gerührten Biogasanlagen zur Vergärung von speziell nachwachsenden Rohstoffen (Energiepflanzen und deren Früchte und Silagen) und tierischen Exkrementen (Gülle, Mist) ist das Kriterium der gleichen Rührerspitzengeschwindigkeit. Damit gilt

$$n \cdot d = \text{konst.}$$

Aus Untersuchungen ist bekannt, dass die Kavernengröße der installierten Rührer (Rührwerke 11) ca. 9% des Fermentervolumens betragen sollte. Bei gleicher Höhe gilt dann:

$$\frac{x \cdot d^2}{D^2} \approx 9\%,$$

wobei *x* für die Anzahl der Rührwerke 11 und *D* für den Behälterdurchmesser (Seitenwandung 5) stehen.

Aus Erfahrungswerten ist weiterhin bekannt, dass ein 3 m-Rührwerk mit einem 10 kW-Motor Gärsubstrate mit bis zu 15% Trockenrückstand (Bei der Vergärung von Nawaro) aus den Biogasanlagen mit einer Drehzahl von 8 rpm sicher durchmischen kann. Die Ausgangskombination wird im Folgenden mit $d_0$, $P_0$ und $n_0$ bezeichnet. Für einen Behälter mit einem beliebigen Durchmesser $D_1$, der drei erfindungsgemäße Rührwerke erhalten soll, gilt dann:

$$d_1 = D_1 \cdot \sqrt{\frac{9\%}{3}}$$

$$n_1 = n_0 \cdot \frac{d_0}{d_1}$$

$$P_1 = P_0 \cdot \frac{d_1^2}{d_0^2}$$

Der Zusammenhang zwischen Rührwerksdurchmesser, Drehzahl und Wellenleistung als Funktion des Behälterdurchmessers ist in der nachstehenden Tabelle 2 bei drei Rührwerken beschrieben:

**Tabelle 2 (drei Rührwerke)**

| Behälterdurchmesser $D_1$ [m] | Rührwerksdurchmesser $d_1$ [m] | Drehzahl $n_1$ [rpm] | Wellenleistung $P_1$ [kW] |
|---|---|---|---|
| 12 | 2,1 | 11,5 | 4,8 |
| 13 | 2,3 | 10,7 | 5,6 |
| 14 | 2,4 | 9,9 | 6,5 |
| 15 | 2,6 | 9,2 | 7,5 |
| 16 | 2,8 | 8,7 | 8,5 |
| 17 | 2,9 | 8,2 | 9,6 |
| 18 | 3,1 | 7,7 | 10,8 |
| 19 | 3,3 | 7,3 | 12,0 |
| 20 | 3,5 | 6,9 | 13,3 |
| 21 | 3,6 | 6,6 | 14,7 |
| 22 | 3,8 | 6,3 | 16,1 |
| 23 | 4,0 | 6,0 | 17,6 |
| 24 | 4,2 | 5,8 | 19,2 |
| 25 | 4,3 | 5,5 | 20,8 |
| 26 | 4,5 | 5,3 | 22,5 |
| 27 | 4,7 | 5,1 | 24,3 |

In einer Abwandlung des Auführungsbeispiels, bei welchem fünf Rührwerke 11 anstelle von drei Rührwerken 11 eingesetzt werden, ändern sich die Werte gemäß der nachstehenden Tabelle 3:

**Tabelle 3 (fünf Rührwerke)**

| Behälterdurchmesser $D_1$ [m] | Rührwerksdurchmesser $d_1$ [m] | Drehzahl $n_1$ [rpm] | Wellenleistung $P_1$ [kW] |
|---|---|---|---|
| 12 | 1,6 | 14,9 | 2,9 |
| 13 | 1,7 | 13,8 | 3,4 |
| 14 | 1,9 | 12,8 | 3,9 |
| 15 | 2,0 | 11,9 | 4,5 |
| 16 | 2,1 | 11,2 | 5,1 |
| 17 | 2,3 | 10,5 | 5,8 |
| 18 | 2,4 | 9,9 | 6,5 |
| 19 | 2,5 | 9,4 | 7,2 |
| 20 | 2,7 | 8,9 | 8,0 |
| 21 | 2,8 | 8,5 | 8,8 |
| 22 | 3,0 | 8,1 | 9,7 |
| 23 | 3,1 | 7,8 | 10,6 |
| 24 | 3,2 | 7,5 | 11,5 |
| 25 | 3,4 | 7,2 | 12,5 |
| 26 | 3,5 | 6,9 | 13,5 |
| 27 | 3,6 | 6,6 | 14,6 |

Im laufenden Betrieb werden die Rührwerke 11 so verschaltet, dass sich die Rührwerkskavernen K zweier benachbarter Rührwerke 11 zu einer Kaverne verbinden. Durch Zu- und Abschalten der Rührwerke 11 werden im Fermenter 1 unterschiedliche Bereiche durchströmt. Je nachdem, welche Rührwerke 11 gerade im Betrieb sind, bilden sich unterschiedliche Totzonen im Fermenter 1 aus. Im Gegensatz zu turbulent gemischten Biogasanlagen ist es nicht notwendig, den Fermenter 11 dauerhaft so zu rühren, dass keine Totzonen entstehen. Jedoch sollten die Rührwerke 11 so verschaltet werden, dass jede Totzone durch Kombination verschiedener Rührwerke 11 einmal durchströmt wird. Bis sich eine Rührwerkskaverne K (Fign. 4, 5) voll ausgebildet hat, vergehen im hier relevanten Maßstab der 13 bis 25 m-Behälter ungefähr 5 bis 10 Minuten, wie es aus CFD-Modellierungen bekannt ist. Eine Taktung der Rührwerke von ca. 10 bis 20 Minuten ist in diesem Zusammenhang also sinnvoll. Die Taktung entspricht ca. ein bis zwei Mischzeiten der Rührwerkskaverne. In der Abwandlung des Ausführungsbeispiels mit fünf Rührwerken i bis v werden beispielsweise die folgenden Mischzyklen verwendet:

| | |
|---|---|
| 0:00 | Start Fütterung, Substratzufuhr bei Rührwerk i |
| 0:00 bis 0:12 | Rührwerk i, ii und iv |
| 0:12 bis 0:24 | Rührwerk ii, iii und v |
| 0:24 bis 0:36 | Rührwerk iii, iv und i |
| 0:36 bis 0:48 | Rührwerk iv, v und ii |
| 0:48 bis 1:00 | Rührwerk v, i und iii |

Die Rührzeiten bzw. die Anzahl der aktiven Rührwerke 11 werden so gewählt, dass sich eine mittlere volumendisspierte Leistung von etwa 4 bis 8 W/m$^3$ einstellt.

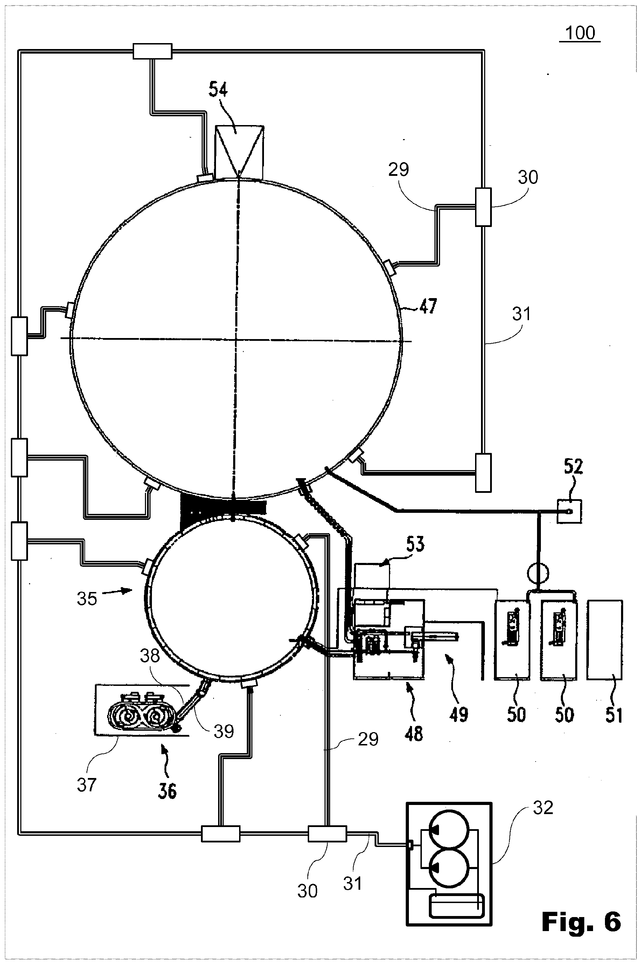

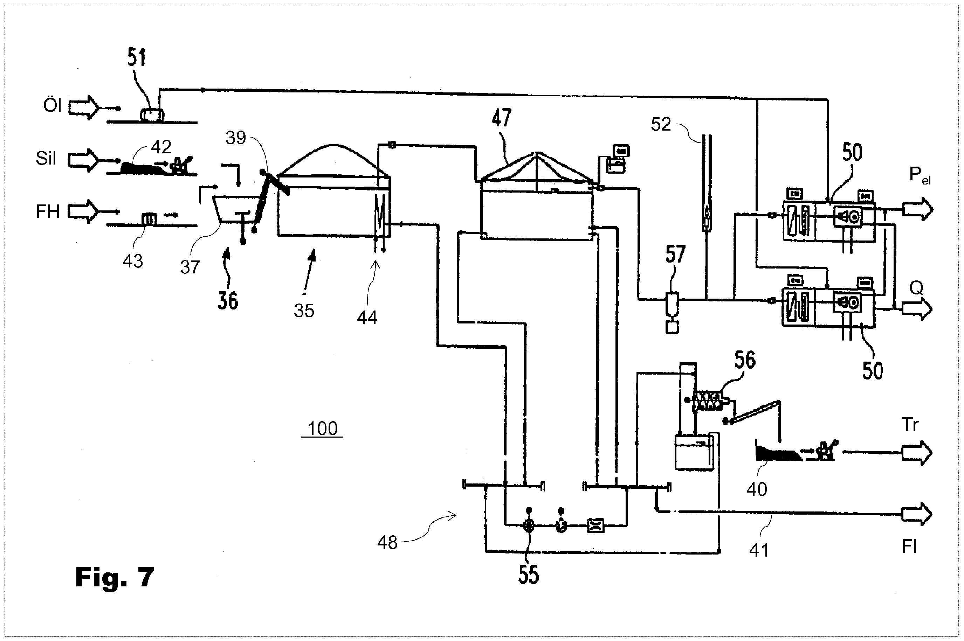

Anhand einer Draufsicht in Fig. 6 und einem Blockschaubild in Fig. 7 in schematischer Seitenansicht wird nun eine Biogasanlage 100 als ein weiteres Ausführungsbeispiel der vorliegenden Erfindung beschrieben.

Gemäß der Darstellung in Fign. 6 und 7 weist die Biogasanlage 100 einen Hauptfermenter 35 und einen Nachgärer 47 auf, wobei der Nachgärer 47 auch als Gasspeicher insgesamt dient. Sowohl der Hauptfermenter 35 als auch der Nachgärer 47 sind Fermenter im Sinne der vorliegenden Anmeldung und können wie der Fermenter 1 des vorherigen Ausführungsbeispiels ausgebildet sein. Ohne Beschränkung der Allgemeinheit weist in der Biogasanlage 100 in diesem Ausführungsbeispiel der Hauptfermenter 35 drei Rührwerke auf und weist der Nachgärer 47 fünf Rührwerke (hier nicht näher dargestellt) auf, die jeweils an einem Rührwerksgestell (hier nicht näher dargestellt) gelagert sind. Die Rührwerke und die Rührwerksgestelle entsprechen den in Fign. 1-5, dort für drei Rührwerke, dargestellten Rührwerken 11 mit Rührwerksgestell 12.

Gemäß der Darstellung in Fig. 6 führen die aus dem Hauptfermenter 35 und dem Nachgärer/Gasspeicher 47 herausgeführten Zuleitungen 29, die im Inneren des Hauptfermenters 35 bzw. des Nachgärers/Gasspeichers 47 mit den als Hydromotoren ausgestalteten Rührantrieben 14 (Fign. 2, 3) verbunden sind, jeweils zu einem Abzweig 30 einer Hydraulikstammleitung 31, die von einer Hydraulikstation 32 versorgt wird. Ohne nähere Darstellung weisen die Abzweige 30 jeweils eine Ventileinrichtung auf, welche von einer Anlagensteuerung (nicht dargestellt) geöffnet oder geschlossen werden können, um die Rührantriebe 14 selektiv anzusteuern. Ferner weisen die Abzweige 30 auch jeweils eine Einrichtung zur Abtrennung des Hüllwassers der Zuleitungen 29 auf, das über getrennte Leitungen (nicht näher dargestellt) zu- und abgeführt wird. Das Hydrauliksystem ist in Fig. 7 aus Gründen der Übersichtlichkeit weggelassen.

Zum Zuführen von Inputstoffen in den Hauptfermenter 35 ist eine Eintragseinrichtung 36 vorgesehen, die einen Vorratsbehälter 37, eine Förderschnecke 38 und einen Förderkanal 39 umfasst. Der Förderkanal 39 mündet im oberen Bereich des Hauptfermenters 35 am oberen Randbereich der Behälterwand in eine Zuführöffnung (vgl. Zuführöffnung 9 in Seitenwandung 5 in Fig. 1).

Der Hauptfermenter 35 wird kontinuierlich mittels der Eintragseinrichtung 36 beschickt. Der Vorratsbehälter 37 der Eintragseinrichtung 36 kann vom Betreiber diskontinuierlich mit Inputmaterial (Silage Sil) von einer Silageschütte 42 und mit einem Fermentationshilfsstoff (FH) aus einem Hilfsstoffbehälter 43 gefüllt werden, wobei die Steuereinrichtung (nicht näher dargestellt) die kontinuierliche Beschickung mittels der Förderschnecke 38 steuert. Mittels der durch die Radarsonde (nicht näher dargestellt) gesteuerten Entnahme erfolgt die Abförderung des abgebauten Materials auch kontinuierlich.

Die typische mittlere hydraulische Verweilzeit des Substrates beträgt etwa 40 Tage im Hauptfermenter 35. Die spezifische Raumbelastung liegt bei 8 kg oTM/m$^3$/d.

Vorzugsweise ist der Hauptfermenter 35 mit einer Heizeinrichtung 44 ausgestattet, die die Temperierung des Substrats im Hauptfermenter 35 erlaubt, so dass eine optimale Gärtemperatur im Hauptfermenter 35 gehalten werden kann.

Die Biogasanlage 100 weist ferner eine Substratabzugsvorrichtung 48, einen Lagerplatz 49 für die festen Gärprodukte, zwei Blockheizkraftwerke 50, einen Öltank 51, eine Biogasnotfackel 52, eine Trafostation 53 und einen Abfüllplatz 54 für die flüssigen Gärprodukte auf.

Im Vorratsbehälter 37 der Eintragseinrichtung 36 wird Silage Sil mit Fermentationshilfsstoffen FH gemischt und diese Mischung wird als Inputstoff dem Hauptfermenter 35 zugeführt. Das im Hauptfermenter 35 erzeugte Biogas wird über eine Biogasleitung (nicht näher bezeichnet) der Gasphase des kombinierten Nachgärers/Gasspeichers 47 zugeführt. Die vergärten Substrate werden über die Substratabzugsvorrichtung 48 entweder dem Nachgärer/Gasspeicher 47 direkt oder über ein Zerkleinerungsaggregat 55 dem Nachgärer/Gasspeicher 47 zugeführt. Der Nachgärer/Gasspeicher 47 ist ein großvolumiger Speicherbehälter mit einer Doppelmembran, wobei sich unterhalb der beiden Membranen das Biogas befindet und unterhalb des Biogases das flüssige Gärprodukt. Das flüssige Gärprodukt kann dem Nachgärer/Gasspeicher 47 und/oder dem Hauptfermenter 35 entnommen und über eine Separationseinheit 56 von seinen Festbestandteilen getrennt werden, die als organischer Dünger (Tr) verwendbar sind, wonach das aufgetrennte flüssige Gärprodukt dem Nachgärer/Gasspeicher 47 zugeführt wird. Somit kann der Trockenrückstand im Nachgärer/Gasspeicher 47 durch die Separationseinheit 56 gesteuert werden. Dieser Dünger (Tr) weist typischerweise einen Trockenrückstand von 30 % bis 35 % auf.

Das im Hauptfermenter 35 abgebaute Substrat kann auch direkt als organischer Dünger verwendet werden. Dies ist ein flüssiger Dünger (Fl) mit einem Trockenrückstand von 10 % bis 15 %.

Das im Nachgärer/Gasspeicher 47 gespeicherte Biogas wird über einen Kondensatabscheider 57 den Blockheizkraftwerken 50 zur Erzeugung von Strom und Wärme zugeführt. Der Öltank 51 ist dazu vorgesehen, die Blockheizkraftwerke 50 mit Zündöl zu versorgen. Weiterhin kann mit einer Notfackel 52 Biogas verbrannt werden, wenn die Blockheizkraftwerke 50 die anfallende Biogasmenge nicht verarbeiten können.

Anstelle oder zusätzlich zu einer sofortigen Verwertung in Wärme und Elektrizität kann auch eine Gasaufbereitungsanlage vorgesehen sein, um das produzierte Biogas zur Zuführung an ein öffentliches Gasversorgungsnetz zu konditionieren. Unter anderem kann dabei ein in einer Biogasanlage erzielbarer CH4-Anteil von ca. 50-55% über eine Reihe von an sich bekannten Verfahrensschritten auf einen Erdgasstandard von ca. 96% CH4-Anteil gebracht werden.

Nun wird ein Verfahren zum Umrüsten einer bestehenden Biogasanlage als ein weiteres Ausführungsbeispiel der vorliegenden Erfindung beschrieben.

Eine Biogasanlage zur Erzeugung von 500 kW besteht aus einem 2.200 m$^3$ Hauptfermenter und einem 4.000 m$^3$ Nachgärer. Die Behälter sind aus dünnen Fertigbetonelementen gefertigt, nachträglich verspannt und können keine zusätzlichen Kräfte oder Momente mehr aufnehmen. Der Ablauf aus dem Nachgärer wird in einem offenen Gärrestlager gelagert. Die Anlage ist mit Tauchmotor-Rührwerken ausgerüstet und turbulent durchmischt. Beide Gärbehälter sind mit Gasspeicherfolien abgedeckt und gasseitig mit einander verbunden. Die Biogasanlage wird mit 7.500 t/a Maissilage und 10.000 t/a Rindergülle gefüttert und produziert Biogas für ein 500 kW-BHKW mit einem elektrischen Wirkungsgrad von 40%. Bei der Entwässerung der Silo- und Verkehrsflächen fallen jährlich 3.000 t Wasser an, die zusätzlich in den Hauptfermenter gepumpt werden. Zur weiteren Verdünnung des Fermenterinhalts werden 5.000 t/a Rezirkulat aus dem Nachgärer in den Hauptfermenter gepumpt.

Die Berechnung der Umsätze und Trockenrückstandskonzentration TR in den Massenströmen erfolgt auf Basis einer kinetischen Modellierung, wie sie in der VDI4630 beschrieben wird. Die Substrate bestehen aus einer schnell abbaubaren Fraktion (Stärke, Hemicellulose) und einer langsam abbaubaren Fraktion (Cellulose), deren Abbau einer Reaktion erster Ordnung folgt. Die Reaktionskonstanten sind $K_1$ = 0,4 d$^{-1}$ für die schnell abbaubare Fraktion f$_1$ und $K_2$ = 0,07 d$^{-1}$ für die langsam abbaubare Fraktion f$_2$.

Es stellt sich im Hauptfermenter ein Trockenrückstand von 7,1% ein, die Verweilzeit beträgt 34,3 d. Der Ablauf aus dem Hauptfermenter wird in den Nachgärer gefördert, wo sich ein Trockenrückstand von 5,8% einstellt. Die Verweilzeit im Nachgärer beträgt 63,4 d. Die sich einstellende Viskosität ist für ein Propellerrührwerk noch zu handhaben und die Behälter können turbulent durchmischt werden. Die Gasproduktion erfolgt zu 85% im Hauptfermenter und zu 15% im Nachgärer. Der Gesamt-Abbaugrad beträgt 96%. Zwar stellt die Gülle fast 50% des gesamten Substratmixes dar, ist aber nur für 11% der erzeugten Biogasmenge verantwortlich. Um den anfallenden Gärrest von 23.046 t/a für 180 Tage zu speichern, ist ein Speichervolumen von mindestens 8900 m$^3$ notwendig und entsprechende Gärrestlager sind zu errichten.

Am Standort der Biogasanlage ändern sich nun die Rahmenbedingungen. Der Rinderstall wird geschlossen und die Maispreise in der Region sind stark gestiegen. Die Rindergülle soll durch Grassilage ersetzt werden, der Maisanteil reduziert werden. Zudem wird die Anlage um ein 250 kW-Satelliten-BHKW erweitert.

Der neue Substratmix lautet: 6000 t/a Mais, 8500 t/a Gras, 3000 t/a Wasser. Es stellt sich ein Trockenrückstand von 14,9% im Hauptfermenter ein, die Verweilzeit beträgt 55,6 d. Im Nachgärer stellt sich ein Trockenrückstand von 12,8% ein, die Verweilzeit beträgt 104 d. Es fallen nur noch 13.976 t/a Gärrest an, zu dessen Lagerung für 180 Tage 6892 m$^3$ Lagervolumen notwendig sind. Der Gesamt-Abbaugrad beträgt 97,6%. Die hohen Trockenrückstandsgehalte in Hauptfermenter und Nachgärer ziehen eine hohe Viskosität nach sich und führen dazu, dass die Behälter nicht mehr mit Propellerrührwerken turbulent durchmischt werden können.

Die Behälter werden stattdessen mit einer erfindungsgemäßen Rührwerksanordnung 12 gemäß Fig. 1-5 und zugehöriger Beschreibung ausgestattet, bei welchem die Momente vollständig in die Beton-Bodenplatte abgeführt werden. Bei der Verwendung von drei Rührwerken müssten die Rührwerke einen Durchmesser von 3,8 m haben und mit ca. 6 U/min betrieben werden. Die installierte Rührwerksleistung sollte mindestens 16 kW betragen (vgl. Tabelle 2). Die installierten Hydraulikaggregate sollen mindestens zwei der drei Rührwerke gleichzeitig betreiben können (32 kW mechanische Leistung an den Wellen).

Bei der Verwendung von fünf Rührwerken können die Rührwerksdurchmesser kleiner gewählt werden. Die Rührer laufen schneller, so dass die Getriebe kleiner gewählt werden können. Auch die Wellenleistung sinkt. Bei der Verwendung von fünf Rührern hätte ein Rührer 3 m Durchmesser, würde mit 8 U/min betrieben werden und müsste einen 10 kW-Motor aufweisen (vgl. Tabelle 3). Die Hydraulikaggregate werden so dimensioniert, dass immer drei von fünf Rührwerken laufen (30 kW mechanische Leistung an den Wellen).

Im Vergleich zum alten Substratmix ergeben sich die folgenden Vorteile:

- Leistung der Anlage steigt von 500 kW auf 750 kW
- Die Verweilzeiten steigen von 34,6 d (Hauptfermenter) und 63,4 d (Nachgärer) auf 55,9 d und 104,5 d
- Abbaugrad steigt von 96,1% auf 97,6%
- Das Lagervolumen für die Lagerung des Gärrest für 180 Tage sinkt von 8.900 m$^3$ auf 6.900 m$^3$, bzw. bei gleichem vorhandenen Gärrestlagervolumen steigt die mögliche Lagerdauer entsprechend an und die Gärreste können effizienter während der optimalen Düngeperiode als Dünger eingesetzt werden.
- Der Anteil der leicht vergärbaren Maissilage kann von 7.500 t/a auf 6.000 t/a reduziert werden

Nachstehend folgt eine Zusammenfassung relevanter Parameter in Tabellenform.

Die nachstehende Tabelle 4 beschreibt zusammenfassend relevante Substrateigenschaften.

**Tabelle 4**

| Substrat | TR [kg/kg] | GV [kg/kg] | $Y_{BG}$ [NM$^3$/t$_{oTM}$] | $X_{CH4}$ [mol/mol] | $W_{hyd}$ [kg/kg] | $\rho$ [kg/Nm$^3$] | $f_1$ [kg/kg] | $f_2$ [kg/kg] |
|---|---|---|---|---|---|---|---|---|
| Mais | 32% | 97% | 750 | 52% | 12% | 1,313 | 50% | 50% |
| Rindergülle | 8% | 85% | 300 | 55% | 12% | 1,276 | 10% | 90% |
| Gras | 35% | 85% | 550 | 55% | 12% | 1,276 | 40% | 60% |
| Wasser | 0% | 0% | - | - | - | - | | |

Die nachstehende Tabelle 5 beschreibt zusammengefasst eine Massenbilanz in einem "Szenario alt" mit turbulent gerührten Fermentern.

**Tabelle 5**

| | FM [t/a] | TM [t/a] | oTM [t/a] | TR [kg/kg] | GV [kg/kg] |
|---|---|---|---|---|---|
| **Hauptfermenter Input:** | | | | | |
| Mais | 7.500 | 2.400 | 2.328 | 32,0% | 97,0% |
| Rindergülle | 10.000 | 800 | 680 | 8,0% | 85,0% |
| Gras | 0 | 0 | 0 | 35,0% | 85,0% |
| Wasser | 3.000 | 0 | 0 | 0,0% | 0,0% |
| Rezirkulat NG | 5.000 | 288 | 235 | 5,8% | 81,5% |
| **Hauptfermenter Output:** | | | | | |
| Biogas | 2.088 | 1.837 | 1.837 | 88,0% | 100,0% |
| Gärrest | 23.412 | 1.651 | 1.406 | **7,1%** | 85,1% |
| **Nachgärer Input:** | | | | | |
| Ablauf VG | 23.412 | 1.651 | 1.406 | 7,1% | 85,1% |
| **Nachgärer Output:** | | | | | |
| Biogas | 366 | 322 | 322 | 88,0% | 100,0% |

(fortgesetzt)

| Nachgärer Output: | | | | | |
|---|---|---|---|---|---|
| Gärrest | 23.046 | 1.329 | 1.083 | 5,8% | 81,5% |
| - Rezirkulat | 5.000 | 288 | 235 | **5,8%** | 81,5% |
| - zum GRL | 18.046 | 1.040 | 848 | 5,8% | 81,5% |

Die nachstehende Tabelle 6 zeigt zusammenfassend eine Massenbilanz in einem "Szenario neu" mit laminar gerührtem Fermenter.

**Tabelle 6**

| | FM [t/a] | TM [t/a] | oTM [t/a] | TR [kg/kg] | GV [kg/kg] |
|---|---|---|---|---|---|
| **Hauptfermenter Input:** | | | | | |
| Mais | 6.000 | 1.920 | 1.862 | 32,0% | 97,0% |
| Rindergülle | 0 | 0 | 0 | 8,0% | 85,0% |
| Gras | 8.500 | 2.975 | 2.529 | 35,0% | 85,0% |
| Wasser | 3.000 | 0 | 0 | 0,0% | 0,0% |
| Rezirkulat NG | 0 | 0 | 0 | 12,8% | 71,9% |
| **Hauptfermenter Output:** | | | | | |
| Biogas | 3.136 | 2.760 | 2.760 | 88,0% | 100,0% |
| Gärrest | 14.364 | 2.135 | 1.631 | **14,9%** | 76,4% |
| **Nachgärer Input:** | | | | | |
| Ablauf VG | 14.364 | 2.135 | 1.631 | 14,9% | 76,4% |
| **Nachgärer Output:** | | | | | |
| Biogas | 388 | 341 | 341 | 88,0% | 100,0% |
| Gärrest | 13.976 | 1.794 | 1.290 | **12,8%** | 71,9% |
| - Rezirkulat | 0 | 0 | 0 | 12,8% | 71,9% |
| - zum GRL | 13.976 | 1.794 | 1.290 | 12,8% | 71,9% |

In nachstehender Tabelle 7 sind die relevanten Parameter noch einmal in kondensierter Form übersichtlich dargestellt.

**Tabelle 7**

| | | | alt | neu |
|---|---|---|---|---|
| **Hauptfermenter** | Volumen | [m$^3$] | 2200 | 2200 |
| | Output | [t/a] | 23.412 | 14.364 |
| | HRT | [d] | 34,3 | 55,9 |
| | Abbau f1 | [kg/kg] | 93,2% | 95,7% |
| | Abbau f2 | [kg/kg] | 70,6% | 79,6% |

(fortgesetzt)

|  |  |  | alt | neu |
|---|---|---|---|---|
| **Nachgärer** | Volumen | [m$^3$] | 4000 | 4000 |
|  | Output | [t/a] | 23.046 | 13.976 |
|  | HRT | [d] | 63,4 | 104,5 |
|  | Abbau f1 | [kg/kg] | 96,2% | 97,7% |
|  | Abbau f2 | [kg/kg] | 81,6% | 88,0% |
| **Gesamt** | Abbau | [kg/kg] | 96,1% | 97,6% |
|  | Abbau f1 | [kg/kg] | 99,6% | 99,8% |
|  | Abbau f2 | [kg/kg] | 93,1% | 95,9% |

Nachfolgend werden noch weitere Beispiele aufgeführt:

Beispiel 1:

Biogasanlage (100), welche wenigstens einen Fermenter (1; 35, 47) aufweist, dadurch gekennzeichnet, dass wenigstens ein Fermenter (1; 35, 47) aufweist:

- einen Fermenterkörper (2)
- eine Substrateintragsvorrichtung (36) zum Einbringen von zu fermentierendem Substrat in einen Innenraum des Fermenterkörpers (2),
- eine Substratabzugsvorrichtung (48) zum Entfernen fermentierten oder teilfermentierten Substrats aus dem Fermenter (1; 35, 47), und
- eine Rührwerksanordnung (3) zum Rühren des Substrats,

und insbesondere nach einem der Ansprüche 10 bis 13 ausgebildet ist.

Beispiel 2:

Biogasanlage wie in Beispiel 1,
dadurch gekennzeichnet, dass
die Biogasanlage (100) wenigstens ein Blockheizkraftwerk (50) aufweist, welches mit Biogas, welches durch die Biogasanlage (100) erzeugt wird, betreibbar ist.

Beispiel 3:

Biogasanlage wie in Beispiel 1 oder 2,
dadurch gekennzeichnet, dass
die Biogasanlage (100) eine Biogasaufbereitungseinrichtung aufweist.

Beispiel 4:

Biogasanlage wie in einem der Beispiele 1 bis 3,
dadurch gekennzeichnet, dass
eine Separationseinheit vorgesehen ist, welche ausgebildet ist, um Fermentationsrückstände und Reststoffe des Betriebs in den oder die Fermenter der Biogasanlage zu rezirkulieren und/oder gesondert bereitzustellen.

Beispiel 5:

Verfahren zur Erzeugung von Biogas durch Fermentation eines Substrats in einem Fermenter (1; 35, 47), der aufweist:

- einen Fermenterkörper (2)

- eine Substrateintragsvorrichtung (36) zum Einbringen von zu fermentierendem Substrat in einen Innenraum des Fermenterkörpers (2),
- eine Substratabzugsvorrichtung (48) zum Entfernen fermentierten oder teilfermentierten Substrats aus dem Fermenter (1; 35, 47), und
- eine Rührwerksanordnung (3) zum Rühren des Substrats,

und insbesondere nach einem der Ansprüche 10 bis 13 ausgebildet ist, und in welchem das Substrat wenigstens teilweise laminar bzw. im Wesentlichen laminar durchmischt wird.

Beispiel 6:

Verfahren wie in Beispiel 5,
dadurch gekennzeichnet, dass
als Bestandteile des Substrats landwirtschaftliche Produkte, insbesondere Energiepflanzen und deren Früchte und/oder Silagen, Gülle und/oder Mist, und/oder Biomüll eingesetzt werden.

Beispiel 7:

Verfahren wie in Beispiel 5 oder 6,
dadurch gekennzeichnet, dass
aus einem fluidführenden, insbesondere wasserführenden, System, welches den/die Rührantrieb/-e (14) umhüllt, aus dem Fermenter (1; 35, 47) ausgeleitetes Fluid hinsichtlich seiner Temperatur überwacht wird, um einen Rührwerksschaden zu detektieren.

Beispiel 8:

Verfahren wie in einem der Beispiele 5 bis 7,
dadurch gekennzeichnet, dass
aus einem fluidführenden, insbesondere wasserführenden, System, welches den/die Rührantrieb/-e (14) umhüllt, aus dem Fermenter (1; 35, 47) ausgeleitetes Wasser auf Verunreinigungen, insbesondere durch Hydrauliköl, untersucht wird.

Beispiel 9:

Verfahren wie in einem der Beispiele 5 bis 8,
dadurch gekennzeichnet, dass
ein fluidführendes, insbesondere wasserführendes, System, welches den/die Rührantrieb/-e (14) umhüllt, hinsichtlich seiner Temperatur geregelt wird.

Beispiel 10:

Verfahren wie in einem der Beispiele 5 bis 9,
dadurch gekennzeichnet, dass
der wenigstens eine Fermenter (1; 35, 47) so betrieben wird, dass mehrere Rührwerke (11) zyklisch derart betrieben werden, dass stets wenigstens ein Rührwerk (11) der mehreren Rührwerke (11) im zyklischen Wechsel stillsteht.

Beispiel 11:

Verfahren wie in einem der Beispiele 5 bis 10,
dadurch gekennzeichnet, dass
das erzeugte Biogas einem Brenner, Blockheizkraftwerk (50) und/oder einer Biogasaufbereitungseinrichtung zugeführt wird.

Beispiel 12:

Verfahren zum Umrüsten eines Fermenters (1; 35, 47) zur Erzeugung von Biogas, mit dem Schritt:

Einbau eines Rührwerksgestells (12), das insbesondere nach einem der Ansprüche 1 bis 3 ausgebildet ist, in einen Fermenterkörper (2) des Fermenters (1; 35, 47).

Beispiel 13:

Verfahren wie in Beispiel 12,
dadurch gekennzeichnet, dass
wenigstens ein weiterer Verfahrensschritt durchgeführt wird, der aus der nachstehenden Gruppe ausgewählt ist:

- Demontage von vorhandenen Rührantrieben,
- Demontage von vorhandenen Rührwellen,
- Demontage von vorhandenen oberen Rührwellenlagern,
- Demontage eines Dachs (6) des Fermenterkörpers (2),
- Demontage von vorhandenen unteren Rührwellenlagern, wenn die Umrüstung den Einbau neuer unterer Rührwellenlager vorsieht,
- Montage von unteren Rührwellenlagern (17) am Fermenterboden (5) oder am Rührwerksgestell (12),
- Montage von oberen Rührwellenlagern (24) am Rührwerksgestell (12),
- Einbau von Rührwellen (13) in den vorgesehenen Rührwellenlagern (24, 17),
- Montage von Rührantrieben (14) am Rührwerksgestell (12),
- Anschluss von Versorgungsleitungen (29),
- Abdichten von Durchlässen der Versorgungsleitungen (29) in der Seitenwandung (5), des Fermenterkörpers (2),
- Montage eines neuen oder des alten Dachs (6)
- Schließen von Durchlässen des Dachs (6), wenn das alte Dach wiederverwendet wird,
- Einbringen von Durchlässen in der Seitenwandung (5) für die Versorgungsleitungen (29).

Im Rahmen dieser Anmeldung wurden folgende Begriffsdefinitionen verwendet:

| | |
|---|---|
| Substrat | Inputmaterial einer Biogasanlage, in Biogas umwandelbarer Trockenrückstand, |
| Gärsubstrat | Inhalt und Ablauf eines Fermenters (Hauptfermenter oder Nachgärer), |
| Trockenrückstand (TR) | Gewichtsanteil der Frischmasse an der Gesamtmasse, definiert nach DIN/EN12880 |
| Glühverlust (GV) | Gewichtsanteil des Trockenrückstandes, der bei 550 °C bis zur Gewichtskonstanz verglüht. Definiert nach DIN/EN12879 |
| Fermenter | Gärbehälter auf einer Biogasanlage zur Erzeugung von Biogas aus organischem Material |
| Hauptfermenter | vorzugsweise beheizbarer Fermenter einer ersten Gärstufe, dem frisches Substrat zugeführt wird |
| Nachgärer | dem Hauptfermenter nachgeschalteter Fermenter zur Erzeugung von Biogas aus Gärsubstrat des Hauptfermenters. Kann unbeheizt sein und mit wechselndem Füllstand betrieben werden |
| Gärrestlager | Speicherbehälter für Gärrest und Gärsubstrat aus Hauptfermenter und Nachgärer |
| Gärrest | Nach der Erzeugung von Biogas verbleibende Reaktionsbrühe, die die Biogasanlage bzw. einen Fermenter verlässt |
| Biogas | das in einem Gärprozess bei ungefähr neutralem pH-Wert entstehende Gas, bestehend aus den beiden Hauptkomponenten $CH_4$ und $CO_2$, sowie Spuren von $H_2S$, $H_2$, Luft und anderen Gasen |
| Nawaro | nachwachsende Rohrstoffe, speziell Energiepflanzen und deren Silagen, bzw. Früchte, sowie Reststoffen die bei der Verwertung von nachwachsenden Rohstoffen anfallen, z.B. Gülle und Mist |
| Laminar | Eigenschaft einer Strömung, die in Schichten aufeinander gleitet und weitestgehend wirbelfrei ist. Wird an Rührorganen durch die Abhängigkeit $P \sim \eta \, n^2 \, d^3$ charakterisiert. |
| Turbulent | Eigenschaft einer wirbelbildenden Strömung. Wird an Rührorganen durch die Abhängigkeit $P \sim n^3 \, d^5 \, \rho$ charakterisiert. |

<div align="center">Liste der Bezugszeichen, Symbole und Abkürzungen</div>

| | | | |
|---|---|---|---|
| 1 | Fermenter | 35 | Hauptfermenter |
| 2 | Fermenterkörper | 36 | Eintragseinrichtung |
| 3 | Rührwerksanordnung | 37 | Vorratsbehälter |
| 4 | Bodenplatte | 38 | Förderschnecke |
| 5 | Seitenwandung | 39 | Förderkanal |
| 6 | Dach | 40 | Trockenlager |
| 7 | Stützsäule | 41 | Ablass |

(fortgesetzt)

| | | | | |
|---|---|---|---|---|
| 8 | Abspannung | 42 | Silageschütte |
| 9 | Zuführöffnung | 43 | Hilfsstoffbehälter |
| 10 | Entnahmeöffnung | 44 | Heizung |
| 11 | Rührwerk | 45 | Aufnahmebereich |
| 12 | Rührwerksgestell | 46 | parallele Strebe |
| 13 | Rührwelle | 47 | Nachgärer/Gasspeicher |
| 14 | Rührantrieb | 48 | Substratabzugsvorrichtung |
| 15 | Rührpaddel | 49 | Lagerplatz |
| 16 | Paddelstange | 50 | Blockheizkraftwerk |
| 17 | unteres Rührwellenlager | 51 | Öltank |
| 18 | Basisplatte | 52 | Biogasnotfackel |
| 19 | Verankerung | 53 | Trafostation |
| 20 | Rahmen | 54 | Abfüllplatz |
| 21 | Rahmenstrebe | 55 | Zerkleinerungsaggregat |
| 22 | Knotenblech (Antriebsaufnahme) | 56 | Separationseinheit |
| 23 | Lageraufnahme | 57 | Kondensatabscheider |
| 24 | oberes Rührwellenlager | 58 | Kopplungselement |
| 25 | Vertikalstrebe | 59 | Findungstrichter |
| 26 | Horizontalstrebe | 100 | Biogasanlage |
| 27 | Winkelstrebe | | |
| 28 | Ankerplatte | | |
| 29 | Zuleitung | | |
| 30 | Abzweig | | |
| 31 | Hydraulikstammleitung | | |
| 32 | Hydraulikstation | | |
| 33 | Stützstrebe | | |
| 34 | Gerüstkranz | | |

A, B, C  Durchnummerierung Substratmischung
CTR  Steuerung
FH  Fermentationshilfsstoffe
FI  Flüssigdünger
GPS  Ganzpflanzensilage
GV  Glühverlust
HRT  Verweildauer
K  Rührwerkskaverne
$K$  Konsistenzfaktor
$K_1, K_2$  Reaktionskonstanten
$K_{MO}$  Metzner-Otto-Konstante des Rührwerks
$Ne$  Newton-Zahl
$P_{el}$  Elektrische Leistung
$P_W$  Wellenleistung,
Q  Wärmeenergie
Sil  Silage
Tr  Trockendünger
TR  Trockenrückstand
$W_{hyd}$  Anteil des für die Hydrolyse benötigten Wassers
$X_{CH4}$  Methananteil
$Y_{BG}$  spezifischer Biogasertrag
Öl  Zündöl

$f_1$  schnell abbaubare Fraktion
$f_2$  langsam abbaubare Fraktion

i, ii, ... v     Durchnummerierung Rührwerke
$m$            Fließindex
$n$            Umdrehungszahl des Rührwerks
$d$            Rührwerksdurchmesser

$\dot{\gamma}$           Scherrate
$\eta$            dynamische Viskosität
$\eta_\infty$          Viskosität bei unendlich hoher Scherrate
$\rho$            Dichte des Mediums
$\tau_0$          Fließgrenze

Die vorstehende Liste ist Bestandteil der Beschreibung.

**Patentansprüche**

**1.** Rührwerksgestell (12) für einen Fermenter (1; 35, 47), wobei das Rührwerksgestell (12) aufweist:

- eine oder mehrere Lageraufnahmen (23), die an dem Rührwerksgestell (12) jeweils zur Aufnahme eines vorzugsweise oberen Rührwellenlagers (24) zur vertikalen bzw. im Wesentlichen vertikalen Lagerung einer Rührwelle (13) ausgebildet ist bzw. sind,
- ein unteres Rührwellenlager (17) am unteren Endbereich der Rührwelle (13), welches lösbar an einem Boden eines Fermenterkörpers (2) anordbar ist, so dass die Rührwelle (13) angehoben werden kann,
- eine oder mehrere Antriebsaufnahmen (22), die jeweils einer der Lageraufnahmen (23) zugeordnet und am Rührwerksgestell (12) zur Aufnahme eines Rührantriebs (14) für die jeweilige Rührwelle (13) ausgebildet ist bzw. sind, und
- eine Verankerungseinrichtung (19), zur Verankerung des Rührwerksgestells (12) relativ zu einem Fermenterkörper (2) eines Fermenters (1; 35, 47), **dadurch gekennzeichnet, dass** die Verankerungseinrichtung (19) derart ausgebildet ist, dass das Rührwerksgestell (12) samt Rührwelle/-n (13) und Rührantrieb/-en (14) vollständig innerhalb eines Fermenterkörpers (2), aufnehmbar ist, und wobei die Verankerungseinrichtung (19) ausgebildet ist, um nur an einer Bodenplatte (4) und/oder an einer Seitenwand (5) eines Fermenterkörpers (2) befestigbar zu sein.

**2.** Rührwerksgestell (12) nach Anspruch 1, wobei der Rührantrieb und insbesondere ein oberes Rührwellenlager oberhalb einer maximalen Substratfüllhöhe angeordnet sind.

**3.** Rührwerksgestell (12) nach Anspruch 1 oder 2,
**dadurch gekennzeichnet, dass**
das Rührwerksgestell (12) einen seitlich offenen Aufnahmebereich (45) aufweist, der zur Aufnahme einer Lageraufnahme (23) und/oder Antriebsaufnahme (22) vorgesehen ist.

**4.** Rührwerksanordnung (3) für einen Fermenter (1; 35, 47),
wobei die Rührwerksanordnung (3) aufweist:

- eines oder mehrere Rührwerke (11) mit jeweils einer Rührwelle (13), einem Rührantrieb (14) zum Antreiben der Rührwelle (13) und einer Lageranordnung (24, 17) zur axialen und radialen Lagerung der Rührwelle (13), und
- ein Rührwerksgestell (12) zur Aufnahme der Rührwelle (13), des Rührantriebs (14) und wenigstens von Teilen der Lageranordnung (24, 17) des Rührwerks (11) bzw. der jeweiligen Rührwerke (11),

**dadurch gekennzeichnet, dass**
das Rührwerksgestell (12) nach einem der Ansprüche 1 bis 3 ausgebildet ist.

**5.** Rührwerksanordnung (3) nach Anspruch 4,
**dadurch gekennzeichnet, dass**
eine Anzahl von Rührpaddeln (15) vorgesehen ist, die an der Rührwelle (13) der jeweiligen Rührwerke (11) vorzugsweise in unterschiedlichen Höhen angebracht sind und die vorzugsweise wenigstens teilweise verstellbar, insbesondere um eine von der Rührwelle (13) weg weisenden Achse schwenkbar, sind.

**6.** Rührwerksanordnung (3) nach Anspruch 4 oder 5,
**dadurch gekennzeichnet, dass**
die Rührwerksanordnung so dimensioniert ist, dass ein Volumen einer Rührwerkskaverne 5% bis 15%, vorzugsweise 8% bis 12%, besonders bevorzugt 9% bis 11% des Netto-Fermentervolumens beträgt.

**7.** Rührwerksanordnung (3) nach einem der Ansprüche 4 bis 6,
**dadurch gekennzeichnet, dass**
ein insbesondere Radialkräfte aufnehmendes oberes Rührwellenlager (24) an dem Rührwerksgestell (12) aufgenommen ist und ein insbesondere Radialkräfte aufnehmendes und vorzugsweise auch Axialkräfte aufnehmendes unteres, insbesondere lösbares Rührwellenlager (17) vorgesehen ist, das zur Anbringung an einer Bodenplatte (4) des Fermenterkörpers (2) ausgebildet ist, so dass die Rührwelle (13) im Fermenterkörper frei stehend anordbar ist.

**8.** Rührwerksanordnung (3) nach einem der Ansprüche 4 bis 7,
**dadurch gekennzeichnet, dass**
der bzw. die Rührantrieb/-e (14) als Elektromotor/-en oder als Hydromotor/-en ausgebildet ist.

**9.** Rührwerksanordnung (3) nach einem der Ansprüche 4 bis 8,
**dadurch gekennzeichnet, dass**
der/die **Rührantrieb/-e** (14) mit einer Abdeckung abgedeckt ist, die gegen die Antriebsaufnahme (22) statisch abgedichtet ist.

**10.** Fermenter (1; 35, 47) zur Herstellung von Biogas, welcher aufweist:

- einen Fermenterkörper (2),
- eine Substrateintragsvorrichtung (36) zum Einbringen von zu fermentierendem Substrat in einen Innenraum des Fermenterkörpers (2),
- eine Substratabzugsvorrichtung (48) zum Entfernen fermentierten oder teilfermentierten Substrats aus dem Fermenter (1; 35, 47), und
- eine Rührwerksanordnung (3) zum Rühren des Substrats,

**dadurch gekennzeichnet, dass**
der Fermenter eine Rührwerksanordnung nach einem der Ansprüche 4 bis 9 aufweist
und wobei das Dach des Fermenterkörpers (2) als ein Foliendach ausgebildet ist.

**11.** Fermenter (1; 35, 47) nach Anspruch 10,
**dadurch gekennzeichnet, dass**
der/die Rührantrieb/-e (14) in einem fluidgeführten, vorzugsweise wassergeführten, System eingehüllt ist/sind, wobei eine Ableitung des fluidführenden Systems aus dem Fermenterkörper (2) vorgesehen ist.

**12.** Fermenter (1; 35, 47) nach Anspruch 11,
**dadurch gekennzeichnet, dass**
der/die Rührantrieb/-e (14) aus einem Hydroantrieb/-en gebildet ist, wobei die Hydraulikleitungen (29) zu den Hydroantrieben in dem fluidgeführten System eingehüllt sind.

**13.** Biogasanlage (100), welche wenigstens einen Fermenter (1; 35, 47) aufweist,
**dadurch gekennzeichnet, dass**
wenigstens ein Fermenter (1; 35, 47) nach einem der Ansprüche 10 bis 12 ausgebildet ist.

**14.** Verfahren zur Erzeugung von Biogas durch Fermentation eines Substrats in einem Fermenter (1; 35, 47), der nach einem der Ansprüche 10 bis 12 ausgebildet ist und in welchem das Substrat wenigstens teilweise laminar bzw. im Wesentlichen laminar durchmischt wird.

**Claims**

**1.** Agitator stand (12) for a fermenter (1; 35, 47), wherein the agitator stand (12) comprises:

- one or several bearing seats (23), which is or are respectively formed on the agitator stand (12) for the receipt of a preferably superior agitator shaft bearing (24) for the vertical or essentially vertical disposition of an agitator shaft (13),
- an inferior agitator shaft bearing (17) at the inferior end zone of the agitator shaft (13), which can be disposed detachably at the ground of the fermentation body (2), so that the agitator shaft (13) can be raised,
- one or more agitator seats (22), which is or are respectively affected to one of the bearing seats (23) and which are formed on the agitator stand (12) for receipt of an agitator (14) for the respective agitator shaft (13), and
- and anchoring installation (19) for anchoring the agitator stand (12) relatively to a fermenter body (2) of a fermenter (1; 35, 47), **characterised in that** the anchoring installation is formed so, that the agitator stand (12) together with agitator shaft(s) (13) and agitator(s) (14) can be received completely within a fermenter body (2), and that the anchoring installation (19 close down is formed so, that it can be attached only at a ground plate (4) and/or on a side wall (5) of a fermenter body (2).

2. Agitator stand (12) according to claim 1, wherein the agitator and in particular the superior agitator shaft bearing is disposed above a maximum substrate fill height.

3. Agitator stand (12) according to claim 1 or 2,
**characterised in that**
the agitator stand (12) comprises a laterally open reception zone (45), which is provided for for the reception of a bearing seat (23) and/or an agitator seed (22).

4. Agitator disposition (3) for a fermenter (1; 35, 47),
wherein the agitator disposition (3) comprises:

- one or more agitators (11) with respectively one agitator shaft (13), one agitator drive (14) for driving the agitator shaft (13) and one bearing disposition (24, 17) for axial and radial bearing of the agitator shaft (13), and
- an agitator stand (12) for receipt of the agitator shaft (13), of the agitator drive (14) and at least a part of the bearing disposition (24, 17) of the agitator (11) or of the respective agitators (11),

**characterised in that**
the agitator stand (12) is formed according to one of claims 1 to 3.

5. Agitator disposition (3) according to claim 4,
**characterised in that**
a number of agitator pedals (15) is provided for, which are attached on the agitator shaft (13) of the respective agitators (11), preferably in different fates, and which preferably are at least partially adjustable, in particular can be rotated around an axis departing from the agitator shaft (13).

6. Agitator disposition (3) according to claim 4 or 5,
**characterised in that**
the agitator disposition is so dimensioned, that the volume of an agitator cavern comprises 5% to 15%, preferably 8% to 12%, particularly preferred 9% to 11% of the fermenter net volume.

7. Agitator disposition (3) according to one of claims 4 to 6,
**characterised in that**
a superior agitator shaft bearing (24) receiving in particular radial forces is received on the agitator stand (12) and an inferior, in particular detachable agitator shaft bearing (17) in particular receiving radial forces and preferably also axial forces is provided for, which is formed for attachment to a base plate (4) of the fermenter body (2), so that the agitator shaft (13) can be disposed standing free in the fermenter body.

8. Agitator disposition (3) according to one of claims 4 to 7,
**characterised in that**
the agitator driver(s) (14) is or are formed as electro motor(s) hydraulic motor(s).

9. Agitator disposition (3) according to one of claims 4 to 8,
**characterised in that**
the agitator driver(s) (14) is or are covered with a cover, which is statically sealed versus the driver bearing (22).

10. Fermenter (1; 35, 47) for the production of biogas, which comprises:

- a fermenter body (2),
- a substrate introduction device (36) for introducing of substrate which is to be fermented into the interior space of the fermenter body (2),
- a substrate deduction device (48) for deducing fermented or partially fermented substrate from the fermenter (1; 35, 47), and
- an agitator disposition (3) for agitating the substrate,

**characterised in that**
the fermenter comprises an agitator disposition according to one of claims 4 to 9 and
wherein the ceiling of the fermenter body (2) is formed as a foil ceiling.

**11.** Fermenter (1; 35, 47) according to claim 10,
**characterised in that**
the agitator driver(s) (14) is or are enrobed in a fluidically managed, preferably aquatically managed system, wherein a deduction of the fluidic system out of the fermenter body (2) is provided for.

**12.** Fermenter (1; 35, 47) according to claim 11,
**characterised in that**
the agitator(s) (14) are formed by one or more hydraulic drivers, wherein the hydraulic conduits (29) to the hydraulic drivers are enrobed in the fluidic system.

**13.** Biogas installation (100) which comprises at least one fermenter (1; 35, 47),
**characterised in that**
at least one fermenter (1; 35, 47) is formed according to one of claims 10 to 12.

**14.** Process for the production of biogas by fermentation of the substrate in a fermenter (1; 35, 47), which is formed according to one of claims 10 to 12 and in which the substrate is mixed at least partially in laminar or essentially laminar fashion.

**Revendications**

**1.** Châssis pour agitateur (12) pour une installation de fermentation (1; 35, 47), dans lequel le châssis pour agitateur (12) comprend :

- un ou plusieurs logements de palier (23), qui est ou qui sont respectivement formé(s) au châssis pour agitateur (12) pour la réception d'un palier préférablement supérieur d'un arbre d'agitation (24) pour la disposition verticale ou essentiellement verticale d'un arbre d'agitation (13),
- un palier d'arbre d'agitation (17) à l'extrémité inférieure de l'arbre d'agitation (13), qui peut être disposé de manière détachable sur le sol d'un corps d'installation de fermentation (2), telle que l'arbre d'agitation (13) peut être soulevé,
- une ou plusieurs prises d'entraînement (22), qui est ou qui sont respectivement affectée(s) à un des logements de palier (23) et formée(s) au châssis d'agitation (12) pour la réception d'un agitateur (14) pour l'arbre d'agitation (13) respectif, et
- un dispositif d'ancrage (19) pour l'ancrage du châssis pour agitateur (12) relativement à un corps d'installation de fermentation (2) d'une installation de fermentation (1; 35, 47), **caractérisé en ce que** le dispositif d'ancrage est formé de telle manière, que le châssis pour agitateur (12) ensemble avec l'arbre ou les arbres d'agitation (13) et l'agitateur ou les agitateurs (14) peut être reçu complètement à l'intérieur d'un corps d'installation de fermentation (2), et que le dispositif d'ancrage (19) est formé pour pouvoir être fixé seulement à une plaque de sol (4) et / ou une paroi latérale (5) d'un corps d'installation de fermentation (2).

**2.** Châssis pour agitateur (12) selon la revendication 1, dans lequel l'agitateur et en particulier un palier d'arbre d'agitation supérieure est disposé au-dessus d'une hauteur de remplissage de substrat maximale.

**3.** Châssis pour agitateur (12) selon la revendication 1 ou 2,
**caractérisé en ce que** le châssis pour agitateur (12) comprend une partie de réception (45) ouverte sur le côté, qui est prévue pour la réception d'un logement de palier (23) et où d'un logement d'agitateur (22).

**4.** Disposition d'agitateurs (3) pour une installation de fermentation (1; 35, 47), dans laquelle la disposition d'agitateurs (3) comprend :

- un ou plusieurs agitateurs (11) avec respectivement un arbre d'agitation (13), un agitateur (14) pour entraîner l'arbre d'agitation (13) et une disposition de paliers (24, 17) pour la disposition axiale et radiale de l'arbre d'agitation (13), et
- un châssis pour agitateur (12) pour la réception de l'arbre d'agitation (13), de l'agitateur (14) et au moins de parties de la disposition de paliers (24, 17) de l'agitateur (11) ou des agitateurs (11) respectifs,

**caractérisée en ce que**
le châssis pour agitateur (12) est formé selon l'une des revendications 1 à 3.

**5.** Disposition d'agitateurs (3) selon la revendication 4,
**caractérisée en ce que**
un nombre de rames d'agitation (15) est prévu, qui sont attachés à l'arbre d'agitation (13) des agitateurs (11) respectifs préférablement à des hauteurs différentes et qui de préférence sont au moins partiellement ajustables, en particulier tournables autour d'un axe s'éloignant de l'arbre d'agitation (13).

**6.** Disposition d'agitateurs (3) selon la revendication 4 ou 5,
**caractérisée en ce que**
la disposition d'agitateur est dimensionnée de telle manière, que le volume d'une caverne d'agitateur comporte 5 % à 15 %, de préférence 8 % à 12 %, particulièrement préféré de 9 % à 11 % du volume net de l'installation de fermentation.

**7.** Disposition d'agitateurs (3) selon l'une quelconque des revendications 4 à 6,
**caractérisée en ce**
**qu'**un palier d'arbre d'agitation supérieur (24) recevant en particulier des forces radiales est reçu au châssis d'agitateur (12) et qu'un palier d'arbre d'agitation inférieure (17) recevant en particulier des forces radiales et de préférence recevant aussi des forces axiales, en particulier détachable, est prévu, lequel est formé pour l'installation à une plaque de sol (4) du corps d'installation de fermentation (2), de telle manière que l'arbre d'agitation (13) peut être disposé librement vertical dans le corps de fermentation.

**8.** Disposition d'agitateurs (3) selon l'une quelconque des revendications 4 à 7,
**caractérisée en ce que**
l'agitateur (14) ou les agitateurs (14) est/sont formé(s) en tant que moteur(s) électrique ou moteur(s) hydraulique.

**9.** Disposition d'agitateurs (3) selon l'une quelconque des revendications 4 à 8,
**caractérisée en ce que**
l'agitateur (14) ou les agitateurs (14) sont couverts d'une couverture statiquement scellée.contre la prise d'entraînement (22).

**10.** Installation de fermentation (1; 35, 47) pour la production de bio gaz, et qui comprend :

- d'un corps d'installation de fermentation (2),
- un dispositif d'alimentation en substrat (36) pour alimenter du substrat à être fermenté dans la chambre intérieure du corps d'installation de fermentation (2),
- un dispositif d'enlèvement de substrat (48) pour l'enlèvement de substrat fermenté ou partiellement fermenté de l'installation de fermentation (1; 35, 47), et
- une disposition d'agitateurs (3) pour l'agitation du substrat,

**caractérisée en ce que**
l'installation de fermentation comprend une disposition d'agitateurs selon l'une des revendications 4 à 9
et dans laquelle la toiture du corps d'installation de fermentation (2) est formée en tant que toiture de pellicule ou de film.

**11.** Installation de fermentation (1; 35, 47) selon la revendication 10,
**caractérisée en ce que**
l'agitateur (14) ou les agitateurs (14) est/sont enrobé(s) dans un système acheminant des fluides, préférablement par acheminement aquatique, dans laquelle une déduction du système acheminant des fluides à partir du corps d'installation de fermentation (2) est prévu.

12. Installation de fermentation (1; 35, 47) selon la revendication 11,
**caractérisée en ce que**
l'agitateur (14) ou les agitateurs (14) est/sont formé(s) en tant qu'agitateur(s) hydraulique, dans laquelle les conduites (29) vers les agitateurs hydrauliques dans le système acheminant des fluides sont enrobés.

13. Installation de biogaz (100), laquelle comprend au moins une installation de fermentation (1; 35, 47),
**caractérisée en ce**
**qu'**au moins une installation de fermentation (1; 35, 47) est formée selon l'une des revendications 10 à 12.

14. Procédé pour la production de biogaz par fermentation d'un substrat dans une installation de fermentation (1; 35, 47) qui est formée selon l'une des revendications 10 à 12 et dans lequel le substrat est au moins mélangé de manière partiellement laminaire ou essentiellement laminaire.

EP 2 878 365 B1

**Fig. 1**

Einzelheit „II"

Fig. 2

Einzelheit „III"

**Fig. 3**

EP 2 878 365 B1

Ansicht „IV"

1

29
19
2
7
4
K
29
12
V
3
19
20
19
29
5

**Fig. 4**

Ansicht „V"

8
6
1

20
12
K
5
19
7
4
11
11

**Fig. 5**

33

**Fig. 6**

**Fig. 7**

Öl

Sil

FH

51

42

39

47

52

50

$P_{el}$

43

37

36

35

44

57

50

Q

100

56

40

Tr

48

55

41

Fl

EP 2 878 365 B1

**Fig. 8**

**Fig. 9**

Fig. 10a

Fig. 10b

**IN DER BESCHREIBUNG AUFGEFÜHRTE DOKUMENTE**

**In der Beschreibung aufgeführte Patentdokumente**

- US 4982373 A
- DE 102005041798 A1
- DE 102010053242 A1
- DE 202006004982 U1
- DE 20111596 U1
- DE 102010014239 A1
- JP 2011088034 A
- WO 2009018971 A1
- WO 001995031353 A1
- DE 202004004101 U1
- DE 202013104292
- EP 2006008488 W
- WO 2007025739 A2